# EUROPEAN PATENT SPECIFICATION

(11) **EP 3 340 799 B1**
(45) Date of publication and mention of the grant of the patent: **27.05.2026**
(21) Application number: 16760323.2
(22) Date of filing: 26.08.2016
(51) Int. Cl.: A01N 63/22, C12N 1/20, A01P 3/00, A01P 21/00

(54) **COMPOSITIONS AND METHODS FOR CONTROLLING PLANT DISEASE**
ZUSAMMENSETZUNG UND VERFAHREN ZUR BEKÄMPFUNG VON PFLANZENBEFALL
COMPOSITIONS ET PROCÉDÉS DE LUTTE CONTRE UNE MALADIE DES PLANTES

(30) Priority: 28.08.2015 US 201562211282 P; 11.04.2016 US 201662320840 P
(43) Date of publication of application: 04.07.2018
(73) Proprietor: Certis USA LLC, Columbia, MD 21046 (US)
(72) Inventor: HAMMER, Philip E., Cary, North Carolina 27513 (US); ROBERTS, Mark A., Chapel Hill, North Carolina 27516 (US); TWIZEYIMANA, Mathias, Durham, North Carolina 27713 (US); RONYAK, Steve, Durham, North Carolina 27707 (US)
(74) Representative: Vossius & Partner Patentanwälte Rechtsanwälte mbB
(86) International application number: PCT/US2016/048969
(87) International publication number: WO 2017/040273

(56) References cited:
- WO-A1-2015/023662
- WO-A1-2015/023662
- WO-A2-2004/024965
- WO-A2-2004/024965
- ROSA RAUDALES ET AL: "Efficacy of Microbial Biopesticides that may be used in Organic Farming", ORGANIC AGRICULTURE, 15 November 2013 (2013-11-15), pages 1 - 6, XP055307870, Retrieved from the Internet <URL:http://articles.extension.org/pages/29382/efficacy-of-microbial-biopesticides-that-may-be-used-in-organic-farming> [retrieved on 20161005]
- VIRGILIO MOJICA-MARÍN ET AL: "Antagonistic activity of selected strains of Bacillus thuringiensis against Rhizoctonia solani of chili pepper", AFRICAN JOURNAL OF BIOTECHNOLOGY, 2 May 2008 (2008-05-02), Victoria Island, pages 1271 - 1276, XP055307800, Retrieved from the Internet <URL:http://www.ajol.info/index.php/ajb/article/download/58661/46989> DOI: 10.5897/AJB08.130
- SHACHI VARDHAN ET AL: "Diversity analysis of biocontrol Bacillus isolated from rhizospheric soil of rice-wheat (Oryza sativa-Triticum aestivum L.) at India", THE JOURNAL OF ANTIBIOTICS, vol. 66, no. 8, 22 May 2013 (2013-05-22), GB, pages 485 - 490, XP055308072, ISSN: 0021-8820, DOI: 10.1038/ja.2013.10
- SUBHASH YADAV ET AL: "Diversity and phylogeny of plant growth-promoting bacilli from moderately acidic soil", JOURNAL OF BASIC MICROBIOLOGY, vol. 51, no. 1, 12 November 2010 (2010-11-12), BERLIN, DE, pages 98 - 106, XP055308101, ISSN: 0233-111X, DOI: 10.1002/jobm.201000098
- APIRAYA THEPSUKHON ET AL: "IDENTIFICATION OF ENDOPHYTIC BACTERIA ASSOCIATED WITH N 2 FIXATION AND INDOLE ACETIC ACID SYNTHESIS AS GROWTH PROMOTERS IN CURCUMA ALISMATIFOLIA GAGNEP.", JOURNAL OF PLANT NUTRITION, vol. 36, no. 9, 29 July 2013 (2013-07-29), US, pages 1424 - 1438, XP055308077, ISSN: 0190-4167, DOI: 10.1080/01904167.2013.793712
- CHRISTOPHER M. JONES ET AL: "Phenotypic and genotypic heterogeneity among closely related soil-borne N2- and N2O-producing Bacillus isolates harboring the nosZ gene", FEMS MICROBIOLOGY ECOLOGY., vol. 76, no. 3, 1 June 2011 (2011-06-01), NL, pages 541 - 552, XP055308075, ISSN: 0168-6496, DOI: 10.1111/j.1574-6941.2011.01071.x

## Description

### FIELD OF THE INVENTION

The invention relates to bacterial strain AIP61892 and populations thereof for controlling plant disease and/or improving an agronomic trait of interest in a plant.

### BACKGROUND

Plant diseases are responsible for significant agricultural losses. Effects can range from mild symptoms to catastrophic plant damage, which can lead to major economic and social consequences. Methods are needed to effectively control plant diseases and the pathogens that cause them.

### SUMMARY

Compositions and methods for controlling plant disease and/or for improving at least one agronomic trait of interest in a plant are provided. Such compositions and methods comprise a population of biocontrol agents comprising a spore, forespore or combinations of cells from bacterial strain AIP61892 or the strain itself that control one or more pathogens that cause plant disease and/or improve at least one agronomic trait of interest. The biological agents or bacterial strain can be used as an inoculant for plants. Methods for growing a plant susceptible to plant disease and methods and compositions for controlling plant disease are also provided. Further provided are methods and compositions of increasing disease resistance in plants. Methods and compositions for improving plant health and/or improving at least one agronomic trait of interest are also provided.

That is, the invention encompasses the following embodiments:
1. A composition comprising:
   (a) bacterial strain AIP61892; and/or
   (b) at least one of a spore, or a forespore, or a combination of cells, forespores and/or spores from AIP61892;
   wherein said bacterial strain, spore, or a forespore, or a combination of cells, forespores and/or spores is present at 10⁵ CFU/gram to 10¹² CFU/gram or at 10⁵ CFU/ml to 10¹² CFU/ml, and wherein an effective amount of said composition improves an agronomic trait of interest of the plant or controls a fungal plant pathogen that causes a fungal plant disease.
2. The composition of embodiments 1, wherein said composition comprises (a) a cell paste, or (b) a wettable powder, or (c) a spray dried formulation, or (d) a stable formulation.
3. An isolated biologically pure culture of a bacterial strain comprising:
   (a) AIP61892; or,
   (b) a spore, or a forespore, or a combination of cells, forespores and/or spores fromAIP61892, preferably,
   wherein said bacterial strain is resistant to a biocide selected from a herbicide, a fungicide, a pesticide, or a crop protection chemical, wherein said culture is produced by growing in the presence of said biocide, wherein said bacterial strain controls a fungal plant pathogen that causes a fungal plant disease.
4. The isolated biologically pure culture of embodiment 3, wherein said biologically pure culture is able to grow in the presence of glyphosate.
5. A bacterial culture comprising
   (a) AIP61892; or,
   (b) a spore, or a forespore, or a combination of cells, forespores and/or spores from AIP61892;
   wherein said bacterial culture has antipathogenic activity against a fingal plant pathogen that causes a fungal plant disease and is able to grow in the presence of glufosinate or an effective amount of said bacterial culture improves an agronomic trait of interest of the plant.
6. A method for growing a plant susceptible to a fungal plant disease or improving a agronomic trait of interest in a plant comprising applying to the plant
   (a) an effective amount of bacterial strain AIP61892; and/or,
   (b) an effective amount of at least one of a spore, or a forespore, or a combination of cells, forespores and/or spores from AIP61892;
   wherein said effective amount comprises at least 10¹² to 10¹⁶ colony forming units (CFU) per hectare, and wherein said effective amount controls a fungal plant pathogen that causes the fungal plant disease or improves the agronomic trait of interest.
7. The method of embodiment 6, wherein said method increases yield of the plant susceptible to the fungal plant disease.
8. The method of embodiment 6 or 7, wherein the fungal plant disease is Asian Soybean Rust (ASR) or damping off complex.
9. The method of any one of embodiments 6-8, wherein the fungal plant pathogen comprises
   one or more fungal pathogens selected from the group consisting of *Botrytis cinerea, Cersospora spp, Cercospora sojina, Cercospora beticola, Alternaria solani, Rhizoctonia solani, Blumeria graminis f sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora* sp., *Phakopsora meibomiae, Phakopsora pachyrizi, Puccinia triticina, Puccinia recondita, Puccinia striiformis, Puccinia graminis, Puccinia spp., Venturia inaequalis, Verticillium spp, Monilinia fructicola, Monilinia lax,* and *Monilinia fructigena,* more preferably,
   wherein said fungal plant pathogen comprises one or more fungal pathogens selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi,* and *Phakopsora meibomiae.*
10. A method of controlling a fungal plant pathogen that causes a fungal plant disease in an area of cultivation comprising:
   (a) planting the area of cultivation with seeds or plants susceptible to the fungal plant disease; and
   (b) applying to the plant susceptible to the fungal plant disease an effective amount of at least one bacterial strain comprising
      i. AIP61892; or,
      ii. a spore, or a forespore, or a combination of cells, forespores and/or spores from AIP61892,
      wherein said effective amount comprises at least 10⁵ to 10¹⁶ colony forming units (CFU) per hectare.
11. The method of embodiment 10, wherein said plant is susceptible to Asian Soybean Rust (ASR) or damping off complex.
12. The method of embodiment 10 or 11, wherein the fungal plant pathogen comprises
   one or more fungal pathogens selected from the group consisting of *Botrytis cinerea, Cersospora spp, Cercospora sojina, Cercospora beticola, Alternaria solani, Rhizoctonia solani, Blumeria graminis f sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora* sp., *Phakopsora meibomiae, Phakopsora pachyrizi, Puccinia triticina, Puccinia recondita, Puccinia striiformis, Puccinia graminis, Puccinia spp., Venturia inaequalis, Verticillium spp, Monilinia fructicola, Monilinia lax,* and *Monilinia fructigena,* more preferably,
   wherein the fungal plant pathogen comprises one or more fungal pathogens selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi,* and *Phakopsora meibomiae.*
13. The method of any one of embodiments 10-12, wherein said method further comprises applying an effective amount of a biocide, wherein said effective amount of the biocide selectively controls an organism of interest while not significantly damaging the crop, preferably,
   wherein the bacterial strain and the biocide are applied simultaneously, or,
   wherein the bacterial strain and the biocide are applied sequentially.
14. The method of embodiment 13, wherein the biocide is a fungicide.
15. A method of making a modified bacterial strain having increased resistance to a biocide of interest, the method comprising:
   (a) providing a population of a bacterial strain comprising AIP61892, wherein said bacterial strain is susceptible to the biocide of interest;
   (b) culturing said bacterial strain in the presence of the biocide of interest; and,
   (c) selecting a modified bacterial strain having an increased resistance to said biocide of interest, wherein the modified bacterial strain retains the ability to control one or more fungal plant diseases and/or control one or more fungal plant pathogens.
16. The method of embodiment 15, wherein said culturing comprises increasing the concentration of the biocide over time.
17. The method of embodiment 15 or 16, wherein said biocide is glyphosate or glufosinate.
18. A method of treating or preventing a fungal plant disease comprising applying to a plant having a fungal plant disease or at risk of developing a fungal plant disease an effective amount of a bacterial strain comprising:
   (a) AIP61892; and/or
   (b) at least one of a spore or a forespore, or a combination of cells, forespores and/or spores from AIP61892,
   wherein said effective amount comprises at least 10¹² to 10¹⁶ CFU per hectare, and wherein said bacterial strain controls a fungal plant pathogen that causes the fungal plant disease.
19. The method of embodiment 18, wherein the bacterial strain treats or prevents one or more plant diseases comprise one or more fungal plant diseases, preferably, wherein the one or more fungal plant diseases comprise Asian Soybean Rust (ASR) or damping off complex.
20. The method of embodiment 18 or 19, wherein the bacterial strain controls one or more fungal plant pathogens.
21. The method of embodiment 20, wherein the one or more fungal plant pathogens
   are selected from the group consisting of *Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora* sp., *Phakopsora meibomiae, Phakopsora pachyrizi, Puccinia triticina, Puccinia recondita, Puccinia striiformis, Puccinia graminis, Puccinia spp., Venturia inaequalis, Verticillium spp, Monilinia fructicola, Monilinia lax,* and *Monilinia fructigena,* more preferably,
   wherein the one or more fungal pathogens selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi,* and *Phakopsora meibomiae.*

### BRIEF DESCRIPTION OF FIGURES

FIG. 1 shows inhibition of Asian soybean rust (ASR) development on whole plant in growth chambers by different bacterial strains. Fungicide azoxystrobin and (+) control strain were added as positive controls while AFS006 and inoculated control were negative controls.
FIG. 2 shows the number of germinated seedlings (stand count) per Hectar by bacterial strains AIP061892 and AIP079428. This figure demonstrates that AIP061892 and AIP079428 produced about a 2-fold increase in germination over *Pythium* inoculated control.
FIG. 3 shows the number of germinated seedlings (stand count) per Hectar by bacterial strain AIP061892 and AIP079428. This figure demonstrates that AIP061892 produced a 50% recovery in germination over *Rhizoctonia solani* inoculated control.

### DETAILED DESCRIPTION

### I. Overview

Compositions and methods for improving at least one agronomic trait of interest and/or improving plant health and/or for controlling one or more plant diseases are provided

### II. Bacterial Strains

Various biocontrol agents or bacterial strains are provided which can be used to control one or more plant disease and/or improve at least one agronomic trait of interest and/or improve plant health, but the strain forming part of the invention is AIP61892 (a *Bacillus subtilus subsp. Subtilus* strain). Cell populations comprising AIP61892 are provided, as well as, populations of spores from this strain.

Thus, various bacterial strains and/or the pesticidal compositions provided herein comprise as an active ingredient (a) a cell population comprising AIP61892, or (b) a population of spores formed from AIP61892. The following list is of other strains, but only strain AIP61892 forms part of the invention.

AIP27511 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67082.

AIP35174 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67084.

AIP25773 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67085.

AIP15251 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67083.

AIP61892 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67089.

AIP79428 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67081.

AIP14931 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67088.

AIP39589 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67087.

AIP36895 was deposited with the Patent Depository of the National Center for Agricultural Utilization Research Agricultural Research Service, U.S. Department of Agriculture, 1815 North University Street, Peoria, Illinois 61604 U.S.A. on August 6, 2015 and assigned NRRL No. B-67086

Each of the deposits identified above will be maintained under the terms of the Budapest Treaty on the International Recognition of the Deposit of Microorganisms for the Purposes of Patent Procedure. Each deposit was made merely as a convenience for those of skill in the art and is not an admission that a deposit is required under 35 U.S.C. §112.

The term "isolated" encompasses a bacterium, spore, or other entity or substance, that has been (1) separated from at least some of the components with which it was associated when initially produced (whether in nature or in an experimental setting), and/or (2) produced, prepared, purified, and/or manufactured by the hand of man. Isolated bacteria may be separated from at least about 10%, about 20%, about 30%, about 40%, about 50%, about 60%, about 70%, about 80%, about 90%, or more of the other components with which they were initially associated.

As used herein, a substance is "pure" if it is substantially free of other components. The terms "purify," "purifying" and "purified" refer to a bacterium, spore, or other material that has been separated from at least some of the components with which it was associated either when initially produced or generated (e.g., whether in nature or in an experimental setting), or during any time after its initial production. A bacterium or spore or a bacterial population or a spore population may be considered purified if it is isolated at or after production, such as from a material or environment containing the bacterium or bacterial population or spore, and a purified bacterium or bacterial population or spore may contain other materials up to about 10%, about 20%, about 30%, about 40%, about 50%, about 60%, about 70%, about 80%, about 90%, or above about 90% and still be considered purified. In some embodiments, purified bacteria or spores and bacterial populations or spore populations are more than about 80%, about 85%, about 90%, about 91%, about 92%, about 93%, about 94%, about 95%, about 96%, about 97%, about 98%, about 99%, or more than about 99% pure. In specific embodiments, a culture of bacteria contains no other bacterial species in quantities to be detected by normal bacteriological techniques.

By "population" is intended a group or collection that comprises two or more (i.e., 10, 100, 1,000, 10,000, 1x10⁶, 1x10⁷, or 1x10⁸ or greater). Various compositions are provided herein that comprise a population of at least bacterial strain AIP61892. In specific embodiments, the population of at least one of the bacterial strain (i.e., AIP61892, or spores or forespores or a combination of cells, forespores and/or spores, formed from AIP61892) comprises a concentration of at least about 10⁵ CFU/ml to about 10¹¹ CFU/ml, about 10⁵ CFU/ml to about 10¹⁰ CFU/ml, about 10⁵ CFU/ml to about 10¹² CFU/ml, about 10⁵ CFU/ml to about 10⁶ CFU/ml, about 10⁶ CFU/ml to about 10⁷ CFU/ml, about 10⁷ CFU/ml to about 10⁸ CFU/ml, about 10⁸ CFU/ml to about 10⁹ CFU/ml, about 10⁹ CFU/ml to about 10¹⁰ CFU/ml, about 10¹⁰ CFU/ml to about 10¹¹ CFU/ml, about 10¹¹ CFU/ml to about 10¹² CFU/ml. In other embodiments, the concentration of the bacterial strain provided herein or active variant thereof comprises at least about 10⁵ CFU/ml, at least about 10⁶ CFU/ml, at least about 10⁷ CFU/ml, at least about 10⁸ CFU/ml, at least about 10⁹ CFU/ml, at least about 10¹⁰ CFU/ml, at least about 10¹¹ CFU/ml, or at least about 10¹² CFU/ml.

A "spore" refers to at least one dormant (at application) but viable reproductive unit of a bacteria species. Non-limiting methods by which spores are formed from each of AIP27511, AIP35174, AIP25773, AIP15251, AIP61892, AIP79428, AIP14931, AIP39589, and AIP36895 (or variants of any thereof) are disclosed elsewhere herein. It is further recognized the populations disclosed herein can comprise a combination of vegetative cells and forepores (cells in an intermediate stage of spore formation); a combination of forespores and spores; or a combination of forespores, vegetative cells and/or spores.

The compositions comprising the bacterial strain (i.e., AIP61892, or a spore or a forespore or a combination of cells, forespores or/and spores, from AIP61892) can further comprise an agriculturally acceptable carrier. The term "agriculturally acceptable carrier" is intended to include any material that facilitates application of a composition to the intended subject (i.e, a plant or plant part susceptible to a plant disease of interest (i.e., Asian Soybean Rust (ASR), or any other disease discloses herein or a plant or plant part for improving an agronomic trait of interest). Carriers used in compositions for application to plants and plant parts are preferably non-phytotoxic or only mildly phytotoxic. A suitable carrier may be a solid, liquid or gas depending on the desired formulation. In one embodiment, carriers include polar or non-polar liquid carriers such as water, mineral oils and vegetable oils. Additional carriers are disclosed elsewhere herein.

In specific disclosures, the bacterial strain is compatible with a biocide. A biocide is a chemical substance that can exert a controlling effect on an organism by chemical or biological means. Biocides include pesticides, such as fungicides; herbicides; insecticides, and other crop protection chemicals. Such compounds are discussed in detail elsewhere herein. A bacterial strain is compatible with a biocide when the bacterial strain is able to survive and/or reproduce in the presence of an effective amount of a biocide of interest. In instances where the bacterial strain is not compatible for a biocide of interest, if desired, methods can be undertaken to modify the bacterial strain to impart the compatibility of interest. Such methods to produce modified bacterial strains include both selection techniques and/or transformation techniques.

By "modified bacterial strain" is intended a population wherein the strain has been modified (by selection and/or transformation) to have one or more additional traits of interest. In some cases the modified bacterial strain comprises AIP61892. In specific disclosures, the modified bacterial strain is compatible with a biocide of interest, including but not limited to, resistance to a herbicide, fungicide, pesticide, or other crop protection chemical. The modified biocide-resistant strains have the same identification characteristics as the original sensitive strain except they are significantly more resistant to the particular herbicide, fungicide, pesticide, or other crop protection chemical. Their identification is readily possible by comparison with characteristics of the known sensitive strain. Thus, isolated populations of modified bacterial strains are provided, but do not form part of the invention.

An increase in resistance to a biocide (i.e., for example, a herbicide, fungicide, pesticide, or other crop protection chemical resistance) refers to the ability of an organism (i.e., bacterial cell or spore) to survive and reproduce following exposure to a dose of the biocide (e.g, herbicide, fungicide, pesticide, or other crop protection chemical) that would normally be lethal to the unmodified organism or would substantially reduce growth of the unmodified organism. In specific disclosures, the increase in resistance to a biocide is demonstrated in the presence of an agriculturally effective amount of the biocide.

In such instances, the modified bacterial strain having resistance to one or more biocides is useful for enhancing the competitiveness of bacterial strains particularly over other microbial agents which are not resistant to herbicides, fungicides, pesticides, or other crop protection chemicals.

Recombinant bacterial strains having resistance to an herbicide, fungicide, pesticide, or other crop protection chemical can be made through genetic engineering techniques and such engineered or recombinant bacterial strains grown to produce a modified population of bacterial strains. A recombinant bacterial strain is produced by introducing polynucleotides into the bacterial host cell by transformation. Methods for transforming microorganisms are known and available in the art. See, generally, Hanahan, D. (1983) Studies on transformation of Escherichia coli with plasmids J. Mol. Biol. 166, 557-77; Seidman, C.E. (1994) In: Current Protocols in Molecular Biology, Ausubel, F.M. et al. eds., John Wiley and Sons, NY; Choi et al. (2006) J. Microbiol. Methods 64:391-397; Wang et al. 2010. J. Chem. Technol. Biotechnol. 85:775-778. Transformation may occur by natural uptake of naked DNA by competent cells from their environment in the laboratory. Alternatively, cells can be made competent by exposure to divalent cations under cold conditions, by electroporation, by exposure to polyethylene glycol, by treatment with fibrous nanoparticles, or other methods well known in the art.

Herbicide resistance genes for use in transforming a recombinant bacterial strain include, but are not limited to, fumonisin detoxification genes (U.S. Patent No. 5,792,931); acetolactate synthase (ALS) mutants that lead to herbicide resistance, in particular the sulfonylurea-type herbicides, such as the S4 and/or Hra mutations; inhibitors of glutamine synthase such as phosphinothricin or basta (e.g., bar gene); and glyphosate resistance (EPSPS gene); gluphosinate, and HPPD resistance (WO 96/38576, U.S. Patent Nos. 6,758,044; 7,250,561; 7,935,869; and 8,124,846), or other such genes known in the art. The bar gene encodes resistance to the herbicide basta, the nptII gene encodes resistance to the antibiotics kanamycin and geneticin, and the ALS-gene mutants encode resistance to the sulfonylurea herbicides including chlorsulfuron, metsulfuron, sulfometuron, nicosulfuron, rimsulfuron, flazasulfuron, sulfosulfuron, and triasulfuron, and the imadizolinone herbicides including imazethapyr, imazaquin, imazapyr, and imazamethabenz.

To identify and produce a modified population of bacterial strains through selection, the bacterial strains are grown in the presence of the herbicide, fungicide, pesticide, or other crop protection chemical as the selection pressure. Susceptible agents are killed while resistant agents survive to reproduce without competition. As the bacterial strains are grown in the presence of the herbicide, fungicide, pesticide, or other crop protection chemical, resistant bacterial strains successfully reproduce and become dominant in the population, becoming a modified population of bacterial strains. Methods for selecting resistant strains are known and include U.S. Patent Nos. 4,306,027 and 4,094,097. The active variant of the bacterial strain comprising a modified population of bacterial strains will have the same identification characteristics as the original sensitive strain except they are significantly more tolerant to the particular herbicide, fungicide, pesticide, or other crop protection chemical. Thus, their identification is readily possible by comparison with characteristics of the known sensitive strain.

Further active variants of the various bacteria provide herein can be identified employing, for example, methods that determine the sequence identity relatedness between the 16S ribosomal RNA, methods to identify groups of derived and functionally identical or nearly identical strains include Multi-locus sequence typing (MLST), concatenated shared genes trees, Whole Genome Alignment (WGA), Average Nucleotide Identity, and MinHash (Mash) distance metric.

In one aspect, the active variants of the bacterial strain AIP61892 include strains that are closely related to any of the disclosed strains by employing the Bishop MLST method of organism classification as defined in Bishop et al. (2009) BMC Biology 7(1)1741-7007-7-3. Thus, an active variant of a bacterial strain disclosed herein includes a bacterial strain that falls within at least a 80%, 85%, 86%, 87%, 88%, 89%, 90%, 91%, 92%, 93%. 94%, 95%, 96%, 97%, 98%, 98.5%, 98.8%, 99%, 99.1%, 99.2%, 99.3%, 99.4%, 99.5%, 99.6%, 99.7%, 99.8%, or 99.9% sequence cut off employing the Bishop method of organism classification as set forth in Bishop et al. (2009) BMC Biology 7(1)1741-7007-7-3. Active variants of the bacteria identified by such methods will retain the ability to improve at least one agronomic trait when applied in an effective amount to a plant, plant part, or an area of cultivation, including for example, reducing plant disease severity and/or reducing plant disease development.

### III. Formulations

The bacteria strain provided herein (i.e., AIP61892or a spore or a forespore or a combination of cells, forespores or/and spores, from AIP61892) can be formulated as a cell paste, wettable powders, a cell pellet, dusts, granules, a slurry, a dry powder, aqueous or oil based liquid products, and the like. Such formulations will comprise the bacteria provided herein in addition to carriers and other agents.

The bacterial strains disclosed herein can be formulated to include at least one or more of an extender, a solvent, spontaneity promoters, carriers, emulsifiers, dispersants, frost protectants, thickeners, and/or adjuvants.

Examples of typical formulations include water-soluble liquids (SL), emulsifiable concentrates (EC), emulsions in water (EW), suspension concentrates (SC, SE, FS, OD), water-dispersible granules (WG), granules (GR) and capsule concentrates (CS); WG; GR; BB; SG; ZC these and other possible types of formulation are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576. The formulations may comprise active agrochemical compounds other than one or more active compounds of the disclosure.

The formulations or application forms of the bacterial strain AIP61892 can comprise, but are not limited to, auxiliaries, such as extenders, solvents, spontaneity promoters, carriers, emulsifiers, dispersants, frost protectants, biocides, solid carriers, surfactants, thickeners and/or other auxiliaries, such as adjuvants. An adjuvant in this context is a component which enhances the biological effect of the formulation, without the component itself having a biological effect. Examples of adjuvants are agents which promote the retention, spreading, attachment to the leaf surface, or penetration.

Non-limiting extenders are, for example, water, polar and nonpolar organic chemical liquids, for example from the classes of the aromatic and non-aromatic hydrocarbons (such as paraffins, alkyl benzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide). If the extender used is water, it is also possible to employ, for example, organic solvents as auxiliary solvents. Essentially, non-limiting liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethylformamide and dimethyl sulphoxide, and also water. In principle it is possible to use any suitable solvent. Non-limiting solvents are, for example, aromatic hydrocarbons, such as xylene, toluene or alkylnaphthalenes, for example, chlorinated aromatic or aliphatic hydrocarbons, such as chlorobenzene, chloroethylene or methylene chloride, for example, aliphatic hydrocarbons, such as cyclohexane, for example, paraffins, petroleum fractions, mineral and vegetable oils, alcohols, such as methanol, ethanol, isopropanol, butanol or glycol, for example, and also their ethers and esters, ketones such as acetone, methyl ethyl ketone, methyl isobutyl ketone or cyclohexanone, for example, strongly polar solvents, such as dimethyl sulphoxide, and water.

Non-limiting examples of suitable carriers include, for example, ammonium salts and ground natural minerals such as kaolins, clays, talc, chalk, quartz, attapulgite, montmorillonite or diatomaceous earth, and ground synthetic minerals, such as finely divided silica, alumina and natural or synthetic silicates, resins, waxes and/or solid fertilizers. Mixtures of such carriers may likewise be used. Carriers suitable for granules include the following: for example, crushed and fractionated natural minerals such as calcite, marble, pumice, sepiolite, dolomite, and also synthetic granules of inorganic and organic meals, and also granules of organic material such as sawdust, paper, coconut shells, maize cobs, and tobacco stalks.

Liquefied gaseous extenders or solvents may also be used. Non-limiting examples are those extenders or carriers which at standard temperature and under standard pressure are gaseous, examples being aerosol propellants, such as halogenated hydrocarbons, and also butane, propane, nitrogen and carbon dioxide. Examples of emulsifiers and/or foam-formers, dispersants or wetting agents having ionic or nonionic properties, or mixtures of these surface-active substances, are salts of polyacrylic acid, salts of lignosulphonic acid, salts of phenolsulphonic acid or naphthalenesulphonic acid, polycondensates of ethylene oxide with fatty alcohols or with fatty acids or with fatty amines, with substituted phenols (preferably alkylphenols or arylphenols), salts of sulphosuccinic esters, taurine derivatives (preferably alkylta urates), phosphoric esters of polyethoxylated alcohols or phenols, fatty acid esters of polyols, and derivatives of the compounds containing sulphates, sulphonates and phosphates, examples being alkylaryl polyglycol ethers, alkylsulphonates, alkyl sulphates, arylsulphonates, protein hydrolysates, lignin-sulphite waste liquors and methylcellulose. The presence of a surface-active substance is advantageous if one of the active compounds and/or one of the inert carriers is not soluble in water and if application takes place in water.

Further auxiliaries that may be present in the formulations and in the application forms derived from them include colorants such as inorganic pigments, examples being iron oxide, titanium oxide, Prussian Blue, and organic dyes, such as alizarin dyes, azo dyes and metal phthalocyanine dyes, and nutrients and trace nutrients, such as salts of iron, manganese, boron, copper, cobalt, molybdenum, and zinc.

Stabilizers, such as low-temperature stabilizers, preservatives, antioxidants, light stabilizers or other agents which improve chemical and/or physical stability may also be present. Additionally present may be foam-formers or defoamers.

Furthermore, the formulations and application forms derived from them may also comprise, as additional auxiliaries, stickers such as carboxymethylcellulose, natural and synthetic polymers in powder, granule or latex form, such as gum arabic, polyvinyl alcohol, polyvinyl acetate, and also natural phospholipids, such as cephalins and lecithins, and synthetic phospholipids. Further possible auxiliaries include mineral and vegetable oils.

There may possibly be further auxiliaries present in the formulations and the application forms derived from them. Examples of such additives include fragrances, protective colloids, binders, adhesives, thickeners, thixotropic substances, penetrants, retention promoters, stabilizers, sequestrants, complexing agents, humectants and spreaders. Generally speaking, the active compounds may be combined with any solid or liquid additive commonly used for formulation purposes.

Suitable retention promoters include all those substances which reduce the dynamic surface tension, such as dioctyl sulphosuccinate, or increase the viscoelasticity, such as hydroxypropylguar polymers, for example.

Suitable penetrants in the present context include all those substances which are typically used in order to enhance the penetration of active agrochemical compounds into plants. Penetrants in this context are defined in that, from the (generally aqueous) application liquor and/or from the spray coating, they are able to penetrate the cuticle of the plant and thereby increase the mobility of the active compounds in the cuticle. This property can be determined using the method described in the literature (Baur et al., 1997, Pesticide Science 51: 131-152). Examples include alcohol alkoxylates such as coconut fatty ethoxylate (10) or isotridecyl ethoxylate (12), fatty acid esters such as rapeseed or soybean oil methyl esters, fatty amine alkoxylates such as tallowamine ethoxylate (15), or ammonium and/or phosphonium salts such as ammonium sulphate or diammonium hydrogen phosphate, for example.

The various compositions and formulations disclosed herein comprise an amount of the bacterial strain AIP61892, or a spore or a forespore or a combination of cells, forespores or/and spores, from AIP61892. Such an amount comprises a concentration of the bacterial strain of at least about 10⁵ CFU/gram to about 10¹¹ CFU/gram, about 10⁵ CFU/gram to about 10¹⁰ CFU/gram, about 10⁵ CFU/gram to about 10¹² CFU/gram, about 10⁵ CFU/gram to about 10⁶ CFU/gram, about 10⁶ CFU/gram to about 10⁷ CFU/gram, about 10⁷ CFU/gram to about 10⁸ CFU/gram, about 10⁸ CFU/gram to about 10⁹ CFU/gram, about 10⁹ CFU/gram to about 10¹⁰ CFU/gram, about 10¹⁰ CFU/gram to about 10¹¹ CFU/gram, or about 10¹¹ CFU/gram to about 10¹² CFU/gram. In other embodiments, the concentration of the bacterial strain comprises at least about 10⁵ CFU/gram, at least about 10⁶ CFU/gram, at least about 10⁷ CFU/gram, at least about 10⁸ CFU/gram, at least about 10⁹ CFU/gram, at least about 10¹⁰ CFU/gram, at least about 10¹¹ CFU/gram, at least about 10¹² CFU/gram, at least about 10⁴ CFU/gram. Such concentrations of the bacterial strain can occur in any formulation type of interest, including, for example in a wettable power, spray dried formulation, or in a cell paste.

Cell pastes and wettable powers and spray dried formulations comprise the bacterial strain AIP61892, or a spore or a forespore or a combination of cells, forespores or/and spores, from AIP61892. The amount of the bacterial strain can comprise a concentration of the bacterial strain of at least about 10⁵ CFU/gram to about 10¹¹ CFU/gram, about 10⁷ CFU/gram to about 10¹⁰ CFU/gram, about 10⁷ CFU/gram to about 10¹¹ CFU/gram, about 10⁶ CFU/gram to about 10¹⁰ CFU/gram, about 10⁶ CFU/gram to about 10¹¹ CFU/gram, about 10¹¹ CFU/gram to about 10¹² CFU/gram, about 10⁵ CFU/gram to about 10¹⁰ CFU/gram, about 10⁵ CFU/gram to about 10¹² CFU/gram, about 10⁵ CFU/gram to about 10⁶ CFU/gram, about 10⁶ CFU/gram to about 10⁷ CFU/gram, about 10⁷ CFU/gram to about 10⁸ CFU/gram, about 10⁸ CFU/gram to about 10⁹ CFU/gram, about 10⁹ CFU/gram to about 10¹⁰ CFU/gram, about 10¹⁰ CFU/gram to about 10¹¹ CFU/gram, or about 10¹¹ CFU/gram to about 10¹² CFU/gram. In some embodiments, the concentration of the bacterial strain comprises at least about 10⁵ CFU/gram, at least about 10⁶ CFU/gram, at least about 10⁷ CFU/gram, at least about 10⁸ CFU/gram, at least about 10⁹ CFU/gram, at least about 10¹⁰ CFU/gram, at least about 10¹¹ CFU/gram, at least about 10¹² CFU/gram, or at least about 10¹³ CFU/gram.

As used herein, a "cell paste" comprises a population of cells that has been centrifuged and/or filtered or otherwise concentrated.

Further provided is a coated seed which comprises a seed and a coating on the seed, wherein the coating comprises at least the bacterial strain AIP61892, or a spore or a forespore or a combination of cells, forespores or/and spores, from AIP61892, wherein said bacterial strain is present on the seed at about 10⁵ CFU/seed to about 10⁷ CFU/seed, at about 10⁴ CFU/seed to about 10⁸ CFU/seed, at about 10⁴ CFU/seed to about 10⁵ CFU/seed, at about 10⁵ CFU/seed to about 10⁶ CFU/seed, at about 10⁶ CFU/seed to about 10⁷ CFU/seed, or at about 10⁷ CFU/seed to about 10⁸ CFU/seed. Various plants of interest are disclosed elsewhere herein.

A seed coating can further comprise at least at least one nutrient, at least one herbicide or at least one pesticide, or at least one biocide. See, for example, US App Pub. 20040336049, 20140173979, and 20150033811.

The various formulations disclosed herein can be stable for at least 30, 40, 50, 60, 70, 80, 90, 100, 125, 150, 200, 225, 250, 275, 300, 325, 350 days, 1.5 years, 2 years or longer. By stable is intened that the formulation retains viable bacteria and/or retains an effective amount of a biologically active bacteria. In one embodiment, the stable formlation retains at least about 1%, about 10%, about 20%, about 30% about 40%, about 50%, about 60%, about 70%, about 80%, or about 90% of CFU/gram in the formulation at a given storage time point when compared to the CFU/gram produced after immediate preparation of the formulation. In another embodiment, the stable formulation retains at least about 30% to 80%, about 50% to about 80%, about 60% to about 70%, about 70% to about 80%, about 40% to about 50%, about 50% to about 60%, about 60% to about 70% of biological activity in the formulation at a given storage time point when compared to the biological activity found in the formulation immediately after production. In another embodiment, the stable formulation at a given storage time point retains at least about 30%, 45%, 50%, 60%, 70%, 80%, 90% of biological activity when compared to the biological activity found in the formulation immediately after production. In still another embodiment, the stable formation retains any combination of the viability and biological activity noted above.

The formulations preferably comprise between 0.00000001 % and 98% by weight of active compound or, with particular preference, between 0.01 % and 95% by weight of active compound, more preferably between 0.5% and 90% by weight of active compound, based on the weight of the formulation.

The active compound content of the application forms prepared from the formulations may vary within wide ranges. The active compound concentration of the application forms may be situated typically between 0.00000001 % and 95% by weight of active compound, preferably between 0.00001 % and 1 % by weight, based on the weight of the application form. Application takes place in a customary manner adapted to the application forms.

Moreover, the bacterial strain provided herein can be mixed with a biocide, such as a fungicide, insecticide, or herbicide to enhance its activity or the activity of the chemical to which it has been added. In some cases, the combination of the bacterial strain and chemical may show synergistic activity where the mixture of the two exceeds that expected from their simple additive effect.

In specific embodiments, the bacterial strain is compatible with agricultural chemicals used to improve performance of biocides. Such agricultural chemicals include safeners, surfactants, stickers, spreaders, UV protectants, and suspension and dispersal aids. Safeners are chemicals that improve or modify the performance of herbicides. Surfactants, spreaders, and stickers are chemicals included in agricultural spray preparations that change the mechanical properties of the spray (for example, by altering surface tension or improving leaf cuticle penetration). UV protectants improve the performance of agricultural biocides by reducing degradation by ultraviolet light. Suspension and dispersal aids improve the performance of biocides by altering their behavior in a spray tank. In instances where the bacterial strain is not compatible with an agricultural chemical of interest, if desired, methods can be undertaken to modify the bacterial strain to impart the compatibility of interest. Such methods to produce modified bacterial strains include both selection techniques and/or transformation techniques.

The bacterial strain provided herein can be used to significantly improve at least one agronomic trait of interest (i.e, reduce disease such as ASR or another fungal pathogen of interest). The bacterial strain provided herein can be used with other pesticides for an effective integrated pest management program. In one embodiment, the biocontrol populations can be mixed with known pesticides in a manner described in WO 94/10845.

Non-limiting examples of compounds and compositions that can be added to the formulation, include but are not limited to, Acetyl tributyl citrate [Citric acid, 2-(acetyloxy)-, tributyl ester]; Agar; Almond hulls; Almond shells; alpha-Cyclodextrin; Aluminatesilicate; Aluminum magnesium silicate [Silicic acid, aluminum magnesium salt]; Aluminum potassium sodium silicate [Silicic acid, aluminum potassium sodium salt]; Aluminum silicate; Aluminum sodium silicate [Silicic acid, aluminum sodium salt]; Aluminum sodium silicate (1:1:1)[Silicic acid (H4SiO4), aluminum sodium salt (1:1:1)]; Ammonium benzoate [Benzoic acid, ammonium salt]; Ammonium stearate [Octadecanoic acid, ammonium salt]; Amylopectin, acid-hydrolyzed, 1-octenylbutanedioate; Amylopectin, hydrogen 1-octadecenylbutanedioate; Animal glue; Ascorbyl palmitate; Attapulgite-type clay; Beeswax; Bentonite; Bentonite, sodian; beta-Cyclodextrin; Bone meal; Bran; Bread crumbs; (+)-Butyl lactate; [Lactic acid, n-butyl ester, (S)]; Butyl lactate [Lactic acid, n-butyl ester]; Butyl stearate [Octadecanoic acid, butyl ester]; Calcareous shale; Calcite (Ca(Co₃)); Calcium acetate; Calcium acetate monohydrate [Acetic acid, calcium salt, monohydrate]; Calcium benzoate [Benzoic acid, calcium salt]; Calcium carbonate; Calcium citrate [Citric acid, calcium salt]; Calcium octanoate; Calcium oxide silicate (Ca₃O(SiO₄)); Calcium silicate [Silicic acid, calcium salt]; Calcium stearate [Octadecanoic acid, calcium salt]; Calcium sulfate; Calcium sulfate dehydrate; Calcium sulfate hemihydrate; Canary seed; Carbon; Carbon dioxide; Carboxymethyl cellulose [Cellulose, carboxymethyl ether]; Cardboard; Carnauba wax; Carob gum [Locust bean gum]; Carrageenan; Caseins; Castor oil; Castor oil, hydrogenated; Cat food; Cellulose; Cellulose acetate; Cellulose, mixture with cellulose carboxymethyl ether, sodium salt; Cellulose, pulp; Cellulose, regenerated; Cheese; Chlorophyll a; Chlorophyll b; Citrus meal; Citric acid; Citric acid, monohydrate; Citrus pectin; Citrus pulp; Clam shells; Cocoa; Cocoa shell flour; Cocoa shells; Cod-liver oil; Coffee grounds; Cookies; Cork; Corn cobs; Cotton; Cottonseed meal; Cracked wheat; Decanoic acid, monoester with 1,2,3-propanetriol; Dextrins; Diglyceryl monooleate [9-Octadecenoic acid, ester with 1,2,3-propanetriol]; Diglyceryl monostearate [9-Octadecanoic acid, monoester with xybis(propanediol)]; Dilaurin [Dodecanoic acid, diester with 1,2,3-propanetriol] Dipalmitin [Hexadecanoic acid, diester with 1,2,3-propanetriol]; Dipotassium citrate [Citric acid, dipotassium salt]; Disodium citrate [Citric acid, disodium salt]; Disodium sulfate decahydrate ; Diatomaceous earth (less than 1% crystalline silica); Dodecanoic acid, monoester with 1,2,3-propanetriol; Dolomite; Douglas fir bark; Egg shells; Eggs; (+)-Ethyl lactate [Lactic acid, ethyl ester, (S)]; Ethyl lactate [Lactic acid, ethyl ester]; Feldspar; Fish meal; Fish oil (not conforming to 40 CFR 180.950) ; Fuller's earth; Fumaric acid; gamma-Cyclodextrin; Gelatins; Gellan gum; Glue (as depolymd. animal collagen); Glycerin [1,2,3-Propanetriol]; Glycerol monooleate [9-Octadecenoic acid (Z)-, 2,3-dihydroxypropyl ester]; Glyceryl dicaprylate [Octanoic acid, diester with 1,2,3-propanetriol]; Glyceryl dimyristate [Tetradecanoic acid, diester with 1,2,3-propanetriol]; Glyceryl dioleate [9-Octadecenoic acid (9Z)-, diester with 1,2,3-propanetriol]; Glyceryl distearate ; Glyceryl monomyristate [Tetradecanoic acid, monoester with 1,2,3-propanetriol]; Glyceryl monooctanoate [Octanoic acid, monoester with 1,2,3-propanetriol]; Glyceryl monooleate [9-Octadecenoic acid (9Z)-, monoester with 1,2,3-propanetriol]; Glyceryl monostearate [Octadecanoic acid, monoester with 1,2,3-propanetriol]; Glyceryl stearate [Octadecanoic acid, ester with 1,2,3-propanetriol]; Granite; Graphite; Guar gum; Gum Arabic; Gum tragacanth; Gypsum; Hematite (Fe₂O₃); Humic acid; Hydrogenated cottonseed oil; Hydrogenated rapeseed oil; Hydrogenated soybean oil; Hydroxyethyl cellulose [Cellulose, 2-hydroxyethyl ether]; Hydroxypropyl cellulose [Cellulose, 2-hydroxypropyl ether]; Hydroxypropyl methyl cellulose [Cellulose, 2-hydroxypropyl methyl ether]; Iron magnesium oxide (Fe₂MgO₄); Iron oxide (Fe₂O₃); Iron oxide (Fe₂O₃); Iron oxide (Fe₃O4); Iron oxide (FeO); Isopropyl alcohol [2-Propanol]; Isopropyl myristate; Kaolin; Lactose; Lactose monohydrate; Lanolin; Latex rubber; Lauric acid; Lecithins; Licorice extract; Lime (chemical) dolomitic; Limestone; Linseed oil; Magnesium carbonate [Carbonic acid, magnesium salt (1:1); Magnesium benzoate; Magnesium oxide; Magnesium oxide silicate (Mg₃O(Si₂O₅)₂), monohydrate; Magnesium silicate; Magnesium silicate hydrate; Magnesium silicon oxide (Mg₂Si₃O₈); Magnesium stearate [Octadecanoic acid, magnesium salt]; Magnesium sulfate; Magnesium sulfate heptahydrate; Malic acid; Malt extract; Malt flavor; Maltodextrin; Methylcellulose [Cellulose, methyl ether]; Mica; Mica-group minerals; Milk; N/A Millet seed; Mineral oil (U.S.P.); 1-Monolaurin [Dodecanoic acid, 2,3-dihydroxypropyl ester]; 1-Monomyristin [Tetradecanoic acid, 2,3-dihydroxypropyl ester]; Monomyristin [Decanoic acid, diester with 1,2,3-propanetriol]; Monopalmitin [Hexadecanoic acid, monoester with 1,2,3-propanetriol]; Monopotassium citrate [Citric acid, monopotassium salt; Monosodium citrate [Citric acid, monosodium salt]; Montmorillonite; Myristic acid; Nepheline syenite; Nitrogen; Nutria meat; Nylon; Octanoic acid, potassium salt; Octanoic acid, sodium salt; Oils, almond; Oils, wheat; Oleic acid; Oyster shells; Palm oil; Palm oil, hydrogenated; Palmitic acid [Hexadecanoic acid]; Paraffin wax; Peanut butter; Peanut shells ; Peanuts; Peat moss; Pectin; Perlite; Perlite, expanded; Plaster of paris; Polyethylene; Polyglyceryl oleate; Polyglyceryl stearate; Potassium acetate [Acetic acid, potassium salt]; Potassium aluminum silicate, anhydrous; Potassium benzoate [Benzoic acid, potassium salt]; Potassium bicarbonate [Carbonic acid, monopotassium salt]; Potassium chloride; Potassium citrate [Citric acid, potassium salt]; Potassium humate [Humic acids, potassium salts]; Potassium myristate [Tetradecanoic acid, potassium salt]; Potassium oleate [9-Octadecenoic acid (9Z)-, potassium salt; Potassium ricinoleate [9-Octadecenoic acid, 12-hydroxy-, monopotassium salt,(9Z,12R)-]; Potassium sorbate [Sorbic acid, potassium salt ]; Potassium stearate [Octadecanoic acid, potassium salt]; Potassium sulfate; Potassium sulfate [Sulfuric acid, monopotassium salt]; 1,2-Propylene carbonate [1,3-Dioxolan-2-one, 4-methyl-]; Pumice; Red cabbage color (expressed from edible red cabbage heads via a pressing process using only acidified water); Red cedar chips; Red dog flour; Rubber; Sawdust; Shale; Silica, amorphous, fumed (crystalline free); Silica, amorphous, precipated and gel; Silica (crystalline free); Silica gel; Silica gel, precipitated, crystalline-free; Silica, hydrate; Silica, vitreous; Silicic acid (H₂SiO₃), magnesium salt (1:1); Soap (The water soluble sodium or potassium salts of fatty acids produced by either the saponification of fats and oils, or the neutralization of fatty acid); Soapbark [Quillaj a saponin]; Soapstone; Sodium acetate [Acetic acid, sodium salt]; Sodium alginate; Sodium benzoate [Benzoic acid, sodium salt]; Sodium bicarbonate; Sodium carboxymethyl cellulose [Cellulose, carboxymethyl ether, sodium salt]; Sodium chloride; Sodium citrate; Sodium humate [Humic acids, sodium salts]; Sodium oleate; Sodium ricinoleate [9-Octadecenoic acid, 12-hydroxy-, monosodium salt, (9Z,12R)-]; Sodium stearate [Octadecanoic acid, sodium salt]; Sodium sulfate; Sorbitol [D-glucitol]; Soy protein; Soya lecithins [Lecithins, soya]; Soybean hulls; Soybean meal; Soybean, flour; Stearic acid [Octadecanoic acid]; Sulfur; Syrups, hydrolyzed starch, hydrogenated; Tetragylceryl monooleate [9-Octadecenoic acid (9Z)-, monoester with tetraglycerol]; Tricalcium citrate [Citric acid, calcium salt (2:3)]; Triethyl citrate [Citric acid, triethyl ester; Tripotassium citrate [Citric acid, tripotassium salt]; Tripotassium citrate monohydrate [Citric acid, tripotassium salt, monohydrate]; Trisodium citrate [Citric acid, trisodium salt]; Trisodium citrate dehydrate [Citric acid, trisodium salt, dehydrate]; Trisodium citrate pentahydrate [Citric acid, trisodium salt, pentahydrate]; Ultramarine blue [C.I. Pigment Blue 29]; Urea; Vanillia; Vermiculite; Vinegar (maximum 8% acetic acid in solution); Vitamin C [L-Ascorbic acid]; Vitamin; Walnut flour; Walnut shells; Wheat; Wheat flour; Wheat germ oil; Whey; White mineral oil (petroleum); Wintergreen oil; Wollastonite (Ca(SiO3)); Wool; Xanthan gum; Yeast; Zeolites (excluding erionite (CAS Reg. No. 66733-21-9)); Zeolites, NaA; Zinc iron oxide; Zinc oxide (ZnO); and Zinc stearate [Octadecanoic acid, zinc salt].

### IV. Methods of Use

The bacterial strain provided herein can be employed with any plant species to improve an agronomic trait of interest. Agonomic traits of interest include any trait that improves plant health or commercial value. Non-limiting examples of agronomic traits of interest including increase in biomass, increase in drought tolerance, thermal tolerance, herbicide tolerance, drought resistance, insect resistance, fungus resistance, virus resistance, bacteria resistance, male sterility, cold tolerance, salt tolerance, increased yield, enhanced nutrient use efficiency, increased nitrogen use efficiency, increased tolerance to nitrogen stress, increased fermentable carbohydrate content, reduced lignin content, increased antioxidant content, enhanced water use efficiency, increased vigor, increased germination efficiency, earlier or increased flowering, increased biomass, altered root-to-shoot biomass ratio, enhanced soil water retention, or a combination thereof. In other instance, the agronomic trait of interest includes an altered oil content, altered protein content, altered seed carbohydrate composition, altered seed oil composition, and altered seed protein composition, chemical tolerance, cold tolerance, delayed senescence, disease resistance, drought tolerance, ear weight, growth improvement, health enhancement, heat tolerance, herbicide tolerance, herbivore resistance, improved nitrogen fixation, improved nitrogen utilization, improved root architecture, improved water use efficiency, increased biomass, increased root length, increased seed weight, increased shoot length, increased yield, increased yield under water-limited conditions, kernel mass, kernel moisture content, metal tolerance, number of ears, number of kernels per ear, number of pods, nutrition enhancement, pathogen resistance, pest resistance, photosynthetic capability improvement, salinity tolerance, stay-green, vigor improvement, increased dry weight of mature seeds, increased fresh weight of mature seeds, increased number of mature seeds per plant, increased chlorophyll content, increased number of pods per plant, increased length of pods per plant, reduced number of wilted leaves per plant, reduced number of severely wilted leaves per plant, and increased number of non-wilted leaves per plant, a detectable modulation in the level of a metabolite, a detectable modulation in the level of a transcript, or a detectable modulation in the proteome relative to a reference plant.

In one non-limiting embodiment, the bacterial strain provided herein can be employed to decrease or reduce the level of a plant pest. "Pests" includes but is not limited to, insects, fungi, bacteria, nematodes, acarids, protozoan pathogens, animal-parasitic liver flukes, but according to the invention relates to controlling fungi. In one non-limiting embodiment, the bacterial strain provided herein can be employed with any plant species susceptible to a plant disease. By "a plant susceptible to a plant disease" is meant that the causative pathogen(s) of the plant disease are able to infect the plant.

Examples of plant species of interest include, but are not limited to, corn (*Zea mays*), *Brassica* sp. (e.g., *B. napus, B. rapa, B. juncea*)*,* particularly those *Brassica* species useful as sources of seed oil, alfalfa (*Medicago sativa*)*,* rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta*), coffee (*Coffea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*), guava (*Psidium guajava*), mango (*Mangifera indica*), olive (*Olea europaea*), papaya (*Carica papaya*), grape (*Vitus* spp.), strawberry (*Fragaria x ananassa*), cherry (*Prunus* spp.), apple (*Malus domestica*), orange (*Citrus* × *sinensis*)₌ cashew (*Anacardium occidentale*), macadamia (*Macadamia integrifolia*), almond (*Prunus amygdalus*), sugar beets (*Beta vulgaris*), sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (C. *sativus*), cantaloupe (C. *cantalupensis*), and musk melon (C. *melo*). Ornamentals include azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum.

Conifers that may be employed in practicing the present disclosure include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*). In specific embodiments, plants of the present disclosure are crop plants (for example, corn, alfalfa, sunflower, *Brassica,* soybean, cotton, safflower, peanut, sorghum, wheat, millet, tobacco, etc.). In other embodiments, corn and soybean plants are optimal, and in yet other embodiments corn plants are optimal.

Other plants of interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica,* maize, alfalfa, palm, coconut, etc. Leguminous plants include beans, peas, and dry pulses. Beans include guar, locust bean, fenugreek, soybean, garden beans, cowpea, mungbean, lima bean, fava bean, lentils, chickpea, etc.

### A. Non-limiting Plant Pests

Examples of plant diseases which can be treated or reduced or prevented include, but are not limited to, plant diseases caused by fungi, viruses or viroids, bacteria, insects, nematodes, protozoa, but according to the invention, relates to treating, reducing or preventing fungi. Examples of fungal plant diseases include, but are not limited to, Asian Soybean Rust (ASR), gray mold, leaf spot, Frogeye Leaf Spot, Early Blight, Damping off complex, Brown Patch, black scurf, root rot, belly rot, sheath blight, Powdery Mildew, Anthracnose leaf spot, Downy Mildew, Pythium Blight, Late Blight, Fusarium Head Blight, sudden death syndrome (SDS), Fusarium Wilt, Corn Stalk Rot, Brown Rust, Black Rust, Yellow Rust, Wheat Rust, Rust, Apple Scab, Verticillium Wilt, Fire Blight, and Brown Rot, to name a few.

The methods and compositions disclosed herein can be used to control one or more fungal pathogens. A fungal pathogen can be, but is not limited to, a fungus selected from the group consisting of *Botrytis spp., Botrytis cinerea, Cersospora spp, Cercospora sojina, Cercospora beticola, Alternaria spp., Alternaria solani, Rhizoctonia spp., Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum spp, Colletotrichum gloeosporiodes, Discula fraxinea, Mycosphaerella spp., Phomopsis spp., Plasmopara viticola, Pseudoperonospora cubensis, Peronospora belbahrii, Bremia lactucae, Peronospora lamii, Plasmopara obduscens, Pythium spp., Pythium cryptoirregulare, Pythium aphanidermatum, Pythium irregulare, Pythium sylvaticum, Pythium myriotylum, Pythium ultimum, Phytophthora spp., Phytophthora capsici, Phytophthora nicotianae, Phytophthora infestans, Phytophthora tropicalis, Phytophthora sojae, Fusarium spp., Fusarium graminearum, Fusarium solani, Fusarium oxysporum, Fusarium graminicola, Gibberella zeae, Colletotrichum graminicola, Penicillium spp., Phakopsora* sp., *Phakopsora meibomiae, Phakopsora pachyrizi, Puccinia triticina, Puccinia recondita, Puccinia striiformis, Puccinia graminis, Puccinia spp., Sclerotium spp., Sclerotinia ssp., Venturia inaequalis, Verticillium spp, Erwinia amylovora, Monilinia spp., Monilinia fructicola, Monilinia lax,* and *Monilinia fructigena.*

In some embodiments, the fungal pathogen is selected from the group consisting of *Botrytis cinerea, Cercospora sojina, Alternaria solani, Rhizoctonia solani, Erysiphe necator, Podosphaera xanthii, Colletotrichum cereal, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi,* and *Venturia*

### inaequalis

In further embodiments, the fungal pathogen is *Phakopsora* sp., including *Phakopsora pachyrhizi* and/or *Phakopsora meibomiae.*

In further embodiments, the bacterial strain (i.e., AIP61892, or a spore, or a forespore or a combination of cells, forespores and/or spores, from AIP61892) control at least one, two, three, four, five, or more of the fungal diseases and/or fungal pathogens described herein.

In further embodiments, the bacterial strain (i.e., AIP61892, or a spore, or a forespore or a combination of cells, forespores and/or spores, from AIP61892) control at least one, two, three, four, five, or more fungal diseases selected from the group consisting of Asian Soybean Rust, gray mold, leaf spot, Frogeye Leaf Spot, Early Blight, Damping off complex, Brown Patch, black scurf, root rot, belly rot, sheath blight, Powdery Mildew, Anthracnose leaf spot, Downy Mildew, Pythium Blight, Late Blight, Fusarium Head Blight, SDS, Fusarium Wilt, Corn Stalk Rot, Brown Rust, Black Rust, Yellow Rust, Wheat Rust, Rust, Apple Scab, Verticillium Wilt, Fire Blight, and Brown Rot.

In further embodiments, the bacterial strain (i.e., AIP61892, or a spore, or a forespore or a combination of cells, forespores and/or spores, from AIP61892, or an active variant of any thereof) control at least one, two, three, four, five, or more fungal diseases selected from the group consisting of Asian Soybean Rust, gray mold, Frogeye Leaf Spot, Early Blight, Damping off complex, Brown Patch, Powdery Mildew, Anthracnose leaf spot, Downy Mildew, Pythium Blight, Late Blight, Fusarium Head Blight, SDS, and Apple Scab.

In further embodiments, the bacterial strain (i.e., AIP61892, or a spore, or a forespore or a combination of cells, forespores and/or spores, from AIP61892) control at least one, two, three, four, five, or more fungal pathogens selected from the group consisting of *Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora* sp., *Phakopsora meibomiae, Phakopsora pachyrizi, Puccinia triticina, Puccinia recondita, Puccinia striiformis, Puccinia graminis, Puccinia spp., Venturia inaequalis, Verticillium spp, Erwinia amylovora, Monilinia fructicola, Monilinia lax,* and *Monilinia fructigena.*

In further embodiments, the bacterial strain (i.e., AIP61892, or a spore, or a forespore or a combination of cells, forespores and/or spores, from AIP61892) control at least one, two, three, four, five, or more fungal pathogens selected from the group consisting of *Botrytis cinerea, Cercospora sojina, Alternaria solani, Rhizoctonia solani, Erysiphe necator, Podosphaera xanthii, Colletotrichum cereal, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi,* and *Venturia inaequalisa.*

In further embodiments, the bacterial strain (i.e., AIP61892, or a spore, or a forespore or a combination of cells, forespores and/or spores, from AIP61892) control at least one, two, or all of *Phakopsora.* In further embodiments, the bacterial strain (i.e., AIP61892, or a spore, or a forespore or a combination of cells, forespores and/or spores from AIP61892) control at least one, or all of *Phakopsora pachyrhizi* and/or *Phakopsora meibomiae.* In other methods, the bacterial strain (i.e., AIP61892, or a spore, or a forespore or a combination of cells, forespores and/or spores from AIP61892, or an active variant of any thereof) control *Phakopsora pachyrhizi.*

### B. Methods of Treating or Preventing Plant Disease

Provided herein are methods of treating or preventing a fungal plant disease comprising applying to a plant having a plant disease or at risk of developing a plant disease an effective amount of the bacterial strain AIP61892 wherein the bacterial strain controls a plant pathogen that causes the plant disease. In certain embodiments, the bacterial strain comprises AIP61892; or a spore, or a forespore or a combination of cells, forespores and/or spores from AIP61892. In some embodiments, the effective amount of the bacterial strain or active variant thereof comprises at least about 10¹² to 10¹⁶ CFU per hectare or least about 10⁴ to 10¹⁶ CFU per hectare, or least about 10⁵ to 10¹¹ CFU per hectare.

In some methods, the bacterial strain is an antipathogenic agent that treats or prevents one, two, three, four, five or more plant diseases. In other methods, the bacterial strain is an antifungal agent that treats or prevents one, two, three, four, five or more fungal plant diseases. The bacterial strain can be employed with any plant species susceptible to a plant disease of interest.

Examples of diseases causes by the fungal pathogens described herein are provided in Table 1. Also provided are non-limiting exemplary crop species that are susceptible to the plant diseases caused by the pathogens. For example, Table 1 shows that *Botrytis cinerea* causes gray mold on all flowering crops. Therefore, a bacterial strain that controls *Botrytis cinerea* can be applied to a plant having gray mold or at risk of developing gray mold in order to treat or prevent gray mold in the plant. Similarly, Table 1 shows that *Rhizoctonia solani* causes Damping off complex in corn, Damping off complex in soybean, Brown Patch in turf, and Damping off complex in ornamentals. Therefore, the bacterial strain AIP61892 that controls *Rhizoctonia solani* can be applied to a plant having Damping off complex and/or brown patch or at risk of developing Damping off complex and/or brown patch in order to treat or prevent Damping off complex and/or brown patch in the plant. In yet another example, Table 1 shows that *Colletotrichum cereal, Apiognomonia errabunda, Apiognomonia veneta, Colletotrichum gloeosporiodes, Discula fraxinea* cause Anthracnose leaf spot. Therefore, the bacterial strain AIP61892 that controls one or more of *Colletotrichum cereal, Apiognomonia errabunda, Apiognomonia veneta, Colletotrichum gloeosporiodes, Discula fraxinea* can be applied to a plant having Anthracnose leaf spot or at risk of developing Anthracnose leaf spot in order to treat or prevent Anthracnose leaf spot in the plant.

**Table 1**

| **Causal Pathogen** | **Disease** | **Crop-species** |
|---|---|---|
| Botrytis cinerea | gray mold | all flowering crops |
| Cersospora spp | Leaf Spot | Ornamentals |
| Cercospora sojina | Frogeye leaf spot | Soybeans |
| Cercospora beticola | | beets, spinach, chard |
| Alternaria solani | Early Blight | solanaceous plants |
| Rhizoctonia solani | Damping off complex | Corn |
| Rhizoctonia solani | Damping off complex | Soybean |
| Rhizoctonia solani | Brown Patch | Turf |
| Rhizoctonia solani | Damping off complex | Ornamentals |
| Rhizoctonia solani | black scurf | potato |
| Rhizoctonia solani | root rot | sugar beet |
| Rhizoctonia solani | belly rot | cucurbit |
| Rhizoctonia solani | sheath blight | rice |
| Blumeria graminis f. sp. Tritici | Powdery Mildew | Wheat |
| Erysiphe necator | Powdery Mildew | Grape |
| Podosphaera xanthii | Powdery Mildew | Cucurbit |
| Golovinomyces cichoracearum | Powdery Mildew | Ornamentals |
| Erysiphe lagerstroemiae | Powdery Mildew | Ornamentals |
| Sphaerotheca pannosa | Powdery Mildew | Ornamentals |
| Colletotrichum cereale | Anthracnose leaf spot | Turf/grasses/cereal |
| Apiognomonia errabunda | Anthracnose leaf spot | Turf/grasses/cereal |
| Apiognomonia veneta | Anthracnose leaf spot | Turf/grasses/cereal |
| Colletotrichum gloeosporiodes | Anthracnose leaf spot | Turf/grasses/cereal |
| Discula fraxinea | Anthracnose leaf spot | Turf/grasses/cereal |
| Plasmopara viticola | Downy Mildew | Grape |
| Pseudoperonospora cubensis | Downy Mildew | Cucurbit |
| Peronospora belbahrii | Downy Mildew | Basil |
| Bremia lactucae | Downy Mildew | Lettuce |
| Peronospora lamii | Downy Mildew | Coleus |
| Plasmopara obduscens | Downy Mildew | Impatiens |
| Pythium cryptoirregulare | Damping off complex | Ornamental Plants |
| Pythium aphanidermatum | Pythium Blight/Damping off complex | turf/ornamentals/row crop |
| Pythium irregulare | Damping off complex | turf/ornamentals/row crop |
| Pythium sylvaticum | Damping off complex | turf/ornamentals/row crop |
| Pythium myriotylum | Damping off complex | turf/ornamentals/row crop |
| Pythium ultimum | Pythium Blight/Damping off complex | turf/ornamentals/row crop |
| Phytophthora capsici | | cucurbit/pepper |
| Phytophthora nicotianae | | ornamental plants |
| Phytophthora infestans | Late Blight | solanaceous plant |
| Phytophthora tropicalis | | ornamental plants/peppers/tropical nut trees |
| Phytophthora sojae | | Soybean |
| Fusarium graminearum | Fusarium Head Blight | Cereals-Wheat |
| Fusarium solani | SDS | Soybean |
| Fusarium oxysporum | Fusarium Wilt | Herbaceous Plants |
| Fusarium graminicola | Corn Stalk Rot | Maize |
| Gibberella zeae | Corn Stalk Rot | Maize |
| Colletotrichum graminicola | Corn Stalk Rot | Maize |
| Phakopsora pachyrizi | Asian Soybean Rust | Soybean |
| Puccinia triticina | Brown Rust | Cereals |
| Puccinia recondita | Black Rust | Cereals |
| Puccinia striiformis | Yellow Rust | Cereals |
| Puccinia graminis | Wheat Rust | Cereals |
| Puccinia spp. | Rust | Ornamentals |
| Venturia inaequalis | Apple Scab | Malus |
| Verticillium spp | Verticillium Wilt | All |
| Erwinia amylovora | Fire Blight | Rosacea family |
| Monilinia fracticola | Brown Rot | Stone Fruits |
| Monilinia laxa | Brown Rot | Stone Fruits |
| Monilinia fructigena | Brown Rot | Stone Fruits |

Also provided herein are methods of treating or preventing Asian Soybean Rust (ASR) comprising applying to a plant having ASR or at risk of developing ASR an effective amount of the bacterial strain comprising AIP61892; or a spore, or a forespore or a combination of cells, forespores and/or spores from AIP61892. In certain embodiments, the effective amount of the bacterial strain comprises at least about 10¹² to 10¹⁶ CFU per hectare and wherein the bacterial strain controls a plant pathogen that causes ASR. In one embodiment, an effective amount of the bacterial strain is used as a foliar application on a plant to treat or prevent ASR.

The bacterial strain can be employed with any plant species susceptible to ASR. By "a plant susceptible to Asian Soybean Rust (ASR)" is meant that the causative pathogen(s) of ASR are able to infect the plant. Examples of plant species susceptible to ASR include, but are not limited to, soybean *(Glycine max),* common bean (*Phaseolus vulgaris*), such as green beans and kidney beans, lima beans (*Phaseolus limensis*), butter beans (*Phaseolus lunatus*), cowpeas (*Vigna unguiculata*), pigeon peas (Cajanus cajan), yam beans such as jicama (*Pachyrhizus erosus*). In a specific embodiment, a soybean plant is employed.

As outlined in further detail herein, in specific embodiments, the bacterial strain controls one or more fungi that causes ASR (such as, for example, *Phakopsora*). ASR is caused by one or more fungal pathogens of the genus *Phakopsora.* In non-limiting embodiments, the fungal pathogens that cause ASR are *Phakopsora pachyrhizi* or *Phakopsora meibomiae.* The ASR pathogen is well adapted for long-distance dispersal, because the spores can be readily carried by the wind, making it an ideal means for introduction to new, rust-free regions. The primary means of dissemination are spores, which can be carried by wind or splashed rain. These pathogens are obligate pathogens surviving and reproducing only on live hosts. In cultivated soybean, the first symptoms are light-brown polygonal lesions of 2 to 5 mm on the adaxial leaf surface. These lesions develop into volcano-shaped lesions known as pustules that appear on the abaxial surface of the leaf, where uredospores are produced.

In further embodiments, the bacterial strain controls *Phakopsora pachyrhizi.* In yet further embodiments, the bacterial strain controls *Phakopsora meibomiae.* Various assays to measure such activity are disclosed elsewhere herein.

The term "treat" or "treating" or its derivatives includes substantially inhibiting, slowing, or reversing the progression of a condition, substantially ameliorating symptoms of a condition or substantially preventing the appearance of symptoms or conditions brought about by the pathogen that causes the plant disease.

In the following definitions, the plant pathogens to be controlled according to the invention are fungal pathogens. Control of other pathogens are not according to the invention.

The terms "controlling" and "protecting a plant from a pathogen" refers to one or more of inhibiting or reducing the growth, germination, reproduction, and/or proliferation of a pathogen of interest; and/or killing, removing, destroying, or otherwise diminishing the occurrence, and/or activity of a pathogen of interest. As such, a plant treated with the bacterial strain provided herein may show a reduced disease severity or reduced disease development in the presence of plant pathogens by a statistically significant amount.

The term "prevent" and is variations means the countering in advance of bacterial, fungal, viral, insect or other pest growth, proliferation, infestation, spore germination, and hyphae growth. In this instance, the composition is applied before exposure to the pathogens.

The term "ameliorate" and "amelioration" relate to the improvement in the treated plant condition brought about by the compositions and methods provided herein. The improvement can be manifested in the forms of a decrease in pathogen growth and/or an improvement in the diseased plant height, weight, number of leaves, root system, or yield. In general, the term refers to the improvement in a diseased plant physiological state.

The term "inhibit" and all variations of this term is intended to encompass the restriction or prohibition of bacterial, fungal, viral, nematode, insect, or any other pest growth, as well as spore germination.

The term "eliminate" relates to the substantial eradication or removal of bacteria, fungi, viruses, nematodes, insects, or any other pests by contacting them with the composition of the disclosure, optionally, according to the methods of the disclosure described below.

The terms "delay", "retard" and all variations thereof are intended to encompass the slowing of the progress of bacterial, fungal, viral, nematode, insect, or any other pest growth, and spore germination. The expression "delaying the onset" is interpreted as preventing or slowing the progression of bacterial, fungal, viral, nematodes, insect, or any other pest growth, infestation, infection, spore germination and hyphae growth for a period of time, such that said bacterial, fungal, viral, nematode, insect, or any other pest growth, infestation, infection, spore germination and hyphae growth do not progress as far along in development, or appear later than in the absence of the treatment according to the disclosure.

A plant, plant part, or area of cultivation treated with the bacterial strain may show a reduced disease severity or reduced disease development in the presence of plant pathogens by a statistically significant amount. A reduced disease severity or reduced disease development can be a reduction of about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% when compared to non-treated control plants. In other instances, the plant treated with a bacterial strain may show a reduced disease severity or reduced disease development in the presence of plant pathogen at least about 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or about 100% greater when compared to non-treated control plants. Methods for assessing plant disease severity are known, and include, measuring percentage of diseased leaf area (Godoy et al. (2006) Fitopatol. Bras. 31(1) 63-68 or by measuring uredinia counts (see Example 1).

By "antipathogenic compositions" or "antipathogenic" is intended that the compositions are capable of suppressing, controlling, preventing and/or killing the invading pathogenic organism. In specific embodiments, an antipathogenic composition reduces the disease symptoms resulting from pathogen challenge by a statistically significant amount, including for example, at least about 10% to at least about 20%, at least about 20% to about 50%, at least about 10% to about 60%, at least about 30% to about 70%, at least about 40% to about 80%, or at least about 50% to about 90% or greater. Hence, the methods of the disclosure can be utilized to protect plants from disease, particularly those diseases that are caused by plant pathogens.

Assays that measure antipathogenic activity are commonly known in the art, as are methods to quantitate disease resistance in plants following pathogen infection. See, for example, U.S. Patent No. 5,614,395. Such techniques include, measuring over time, the average lesion diameter, the pathogen biomass, and the overall percentage of decayed plant tissues. For example, a plant either expressing an antipathogenic polypeptide or having an antipathogenic composition applied to its surface shows a decrease in tissue necrosis (*i.e.,* lesion diameter) or a decrease in plant death following pathogen challenge when compared to a control plant that was not exposed to the antipathogenic composition. Alternatively, antipathogenic activity can be measured by a decrease in pathogen biomass. For example, a plant expressing an antipathogenic polypeptide or exposed to an antipathogenic composition is challenged with a pathogen of interest. Over time, tissue samples from the pathogen-inoculated tissues are obtained and RNA is extracted. The percent of a specific pathogen RNA transcript relative to the level of a plant specific transcript allows the level of pathogen biomass to be determined. See, for example, Thomma et al. (1998) Plant Biology 95:15107-15111.

Furthermore, *in vitro* antipathogenic assays include, for example, the addition of varying concentrations of the antipathogenic composition to paper disks and placing the disks on agar containing a suspension of the pathogen of interest. Following incubation, clear inhibition zones develop around the discs that contain an effective concentration of the antipathogenic polypeptide (Liu et al. (1994) Plant Biology 91:1888-1892). Additionally, microspectrophotometrical analysis can be used to measure the *in vitro* antipathogenic properties of a composition (Hu et al. (1997) Plant Mol. Biol. 34:949-959 and Cammue et al. (1992) J. Biol. Chem. 267: 2228-2233).

### C. Methods of Inducing Disease Resistance in Plants and/or for Improving Plant Health and/or Improving an Agonomic Trait of Interest

Compositions and methods for inducing disease resistance in a plant to plant pathogens are also provided. Accordingly, the compositions and methods are also useful in protecting plants against fungal pathogens, viruses, nematodes, and insects, but only fungal pathogens forms part of the invention. Provided herein are methods of inducing disease resistance against a plant pathogen comprising applying to a plant that is susceptible to a plant disease caused by the plant pathogen an effective amount of a bacterial strain AIP61892; or a spore, or a forespore or a combination of cells, forespores and/or spores from AIP61892. In certain embodiments, the bacterial strain promotes a defensive response to the pathogen that causes the plant disease. In some embodiments, the effective amount of the bacterial strain comprises at least about 10¹² to 10¹⁶ CFU per hectare.

A defensive response in the plant can be triggered after applying the bacterial strain to the plant, but prior to pathogen challenge and/or after pathogen challenge of the plant treated with the bacterial strain.

In some methods, the bacterial strain induces resistance to one, two, three, four, five or more fungal plant pathogens described herein.

By "disease resistance" is intended that the plants avoid the disease symptoms that result from plant-pathogen interactions. That is, pathogens are prevented from causing plant diseases and the associated disease symptoms, or alternatively, the disease symptoms caused by the pathogen are minimized or lessened as compared to a control. Further provided are methods of improving plant health and/or improving an agronomic trait of interest comprising applying to a plant an effective amount of the bacterial strain comprising AIP61892; or a spore, or a forespore or a combination of cells, forespores and/or spores from AIP61892. In some embodiments, the effective amount of the bacterial strain provided herein or active variant thereof comprises at least about 10¹² to 10¹⁶ CFU per hectare.

By "improved plant health" is meant increased growth and/or yield of a plant, increased stress tolerance and/or decreased herbicide resistance, to name a few. Increased stress tolerance refers to an increase in the ability of a plant to decrease or prevent symptoms associated with one or more stresses. The stress can be a biotic stress that occurs as a result of damage done to plants by other living organisms such as a pathogen (for example, bacteria, viruses, fungi, parasites), insects, nematodes, weeds, cultivated or native plants. The stress can also be an abiotic stress such as extreme temperatures (high or low), high winds, drought, salinity, chemical toxicity, oxidative stress, flood, tornadoes, wildfires, radiation and exposure to heavy metals. Non-limiting examples of improved agronomic traits are disclosed elsewhere herein. In specific embodiments, an effective amount of the bacterial strain or active variant thereof improves plant health or improves an agronomic trait of interest by a statistically significant amount, including for example, at least about 10% to at least about 20%, at least about 20% to about 50%, at least about 10% to about 60%, at least about 30% to about 70%, at least about 40% to about 80%, or at least about 50% to about 90% or greater.

### D. Methods of Application to a Plant or Plant Part

The bacterial strain is applied in an effective amount. An effective amount of a bacterial strain is an amount sufficient to control, treat, prevent, inhibit the pathogen that causes a plant disease, and/or reduce plant disease severity or reduce plant disease development. In other embodiments, the effective amount of the bacterial strain is an amount sufficient to improve an agronomic trait of interest and/or to promote or increase plant health, growth or yield of a plant susceptible to a disease. The rate of application of the bacterial strain may vary according to the pathogen being targeted, the crop to be protected, the efficacy of the bacterial strain, the severity of the disease, the climate conditions, the agronomic trait of interest to improve, and the like.

Generally, the rate of bacterial strain is 10⁷ to 10¹⁶ colony forming units (CFU) per hectare. In other embodiments, for a field inoculation, the rate of bacterial strain application is 3 x 10⁷ to 1 x 10¹¹ colony forming units (CFU) per hectare. (This corresponds to about 1 Kg to 10kg of formulated material per hectare). In other embodiments, for a field inoculation, the rate of bacterial strain application is 3 x 10⁷ to 1 x 10¹⁶ colony forming units (CFU) per hectare; about 1x10¹² to about 1x10¹³ colony forming units (CFU) per hectare, about 1x10¹³ to about 1x10¹⁴ colony forming units (CFU) per hectare, about 1x10¹⁴ to about 1x10¹⁵ colony forming units (CFU) per hectare, about 1x10¹⁵ to about 1x10¹⁶ colony forming units (CFU) per hectare, about 1x10¹⁶ to about 1x10¹⁷ colony forming units (CFU) per hectare; about 1x10⁴ to about 1x10¹⁴ colony forming units (CFU) per hectare; about 1x10⁵ to about 1x10¹³ colony forming units (CFU) per hectare; about 1x10⁶ to about 1x10¹² colony forming units (CFU) per hectare; about 1x10⁹ to about 1x10¹¹ colony forming units (CFU) per hectare; or about 1x10⁹ to about 1x10¹¹ colony forming units (CFU) per hectare. In other embodiments, for a field inoculation, the rate of bacterial strain application is at least about 1x10⁴, about 1x10⁵, about 1x10⁶, about 1x10⁷, about 1x10⁸, about 1x10⁹, about 1x10¹⁰, about 1x10¹¹, about 1x10¹²1x10¹³, about 1x10¹⁴, 1x10¹⁵, about 1x10¹⁶, or about 1x10¹⁷ colony forming units (CFU) per hectare. In other embodiments, for a field inoculation, the rate of bacterial strain application is at least 1x10⁷ to at least about 1x10¹² CFU/hectare. In specific embodiments, the bacterial strain applied comprises the strain deposited AIP61892, or a spore, or a forespore or a combination of cells, forespores and/or spores from AIP61892.

Any appropriate agricultural application rate for a biocide can be applied in combination with the bacterial strain disclosed herein. Methods to assay for the effective amount of the bacterial strain include, for example, any statistically significant increase in the control of the pathogen or pest targeted by the biocide. Methods to assay for such control are known. Moreover, a statistically significant increase in the control of plant health, yield and/or growth that occurs upon application of an effective amount of the bacterial strain when compared to the plant health, yield and/or growth that occurs when no bacterial strain is applied.

Further provided is a method for controlling or inhibiting the growth of a plant pathogen that causes plant disease by applying a composition comprising the bacterial strain (i.e., AIP61892, or a spore, or a forespore or a combination of cells, forespores and/or spores from AIP61892). By "applying" is intended contacting an effective amount of the bacterial strain to a plant, area of cultivation, seed and/or weed with one or more of the bacterial strain so that a desired effect is achieved. Furthermore, the application of the bacterial strain can occur prior to the planting of the crop (for example, to the soil, the seed, or the plant). In a specific embodiment, the application of the bacterial strain is a foliar application. Therefore, a further embodiment of the disclosure provides a method for controlling or inhibiting the growth of a plant pathogen by applying the population of bacterial strain to an environment in which the plant pathogen may grow. The application may be to the plant, to parts of the plant, to the seeds of the plants to be protected, or to the soil in which the plant to be protected are growing or will grow. Application to the plant or plant parts may be before or after harvest. Application to the seeds will be prior to planting of the seeds.

In some embodiments, an effective amount of the bacterial strain is used as a foliar application to control or inhibit growth of one or more pathogens selected from the group consisting of *Alternaria* spp., *Alternaria solani, Colletotrichum* spp., *Mycosphaerella* spp., *Phomopsis* spp., *Cercospora* spp., *Botrytis* spp., and *Botrytis cinerea.*

In other embodiments, an effective amount of the bacterial strain is applied to the soil in which the plant to be protected are growing or will grow to control or inhibit growth of one or more pathogens selected from the group consisting of *Rhizoctonia spp., Rhizoctonia solani, Fusarium* spp., *Sclerotium spp., Sclerotinia spp., Sclerotinia sclerotiorum, Phytopthora* spp., and *Pythium* spp.

In some embodiments, an effective amount of the bacterial strain is applied to the plant after harvest to control or inhibit growth of one or more pathogens selected from the group consisting of *Monolinia* spp., *Penicillium spp., Botrytis* ssp., and *Botrytis cinerea.*

As used herein, the term plant includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species.

In specific embodiments, the application of the bacterial strain (i.e., AIP61892, or a spore, or a forespore or a combination of cells, forespores and/or spores from AIP61892) is applied to the leaves of a soybean plant. The timing of application can vary depending on the conditions and geographical location. In specific embodiments, the bacterial strain is applied at the R1 (beginning flowering stage) of soybean development or may be applied earlier depending on ASR onset and the disease severity.

In other embodiments, the biocide to a crop, area of cultivation, or field it is intended that one or more of a particular field, plant crop, seed and/or weed is treated with the bacterial strain and one or more biocide so that a desired effect is achieved.

Various methods are provided for controlling a plant pathogen that causes a plant disease in an area of cultivation containing a plant susceptible to the plant disease. The method comprises planting the area of cultivation with seeds or plants susceptible to the plant disease; and applying to the plant susceptible to the disease, the seed or the area of cultivation of the plant susceptible to the plant disease an effective amount of the bacterial strain (i.e., AIP61892, or a forespore or a combination of cells, forespores and/or spores from AIP61892), wherein the effective amount of the bacterial strain controls the plant disease without significantly affecting the crop. In specific embodiments, the effective amount comprises at least about 10¹² to 10¹⁶ colony forming units (CFU) per hectare.

Further provided is a method for growing a plant susceptible to a plant disease. The method comprises applying to a plant susceptible to the disease, a seed, or an area of cultivation of the plant susceptible to the disease an effective amount of a composition comprising the bacterial strain AIP61892; or a spore, or a forespore or a combination of cells, forespores and/or spores from AIP61892. Various effective amounts of bacterial strain are disclosed elsewhere herein and in one, non-limiting example, the effective amount of the bacterial strain comprises at least about 10¹² to 10¹⁶ colony forming units (CFU) per hectare.

Methods for increasing plant yield are provided, but do not form part of the invention. The "yield" of the plant refers to the quality and/or quantity of biomass produced by the plant. By "biomass" is intended any measured plant product. An increase in biomass production is any improvement in the yield of the measured plant product. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase in yield compared to a plant not exposed to the bacterial strain.

A method for increasing yield in a plant is also provided and comprises applying to a crop or an area of cultivation an effective amount of a composition comprising at least one bacterial strain comprising AIP61892, a spore or a forespore or a combination of cells, forespores and/or spores from AIP61892, wherein said effective amount comprises at least about 10¹² to 10¹⁶ colony forming units (CFU) per hectare, and wherein said composition controls a plant pathogen, thereby increasing yield.

As used herein, an "area of cultivation" comprises any region in which one desires to grow a plant. Such areas of cultivations include, but are not limited to, a field in which a plant is cultivated (such as a crop field, a sod field, a tree field, a managed forest, a field for culturing fruits and vegetables, etc.), a greenhouse, a growth chamber, etc.

Further provided is a coated seed which comprises a seed and a coating on the seed, wherein the coating comprises the bacterial strain AIP61892; or a spore, or a forespore or a combination of cells, forespores and/or spores from AIP61892. In certain embodiments, said bacterial strain is present on the seed at about 10⁵ CFU/seed to about 10⁷ CFU/seed, at about 10⁴ CFU/seed to about 10⁸ CFU/seed, at about 10⁴ CFU/seed to about 10⁵ CFU/seed, at about 10⁵ CFU/seed to about 10⁶ CFU/seed, at about 10⁶ CFU/seed to about 10⁷ CFU/seed, or at about 10⁷ CFU/seed to about 10⁸ CFU/seed. The seed coating can be applied to any seed of interest (i.e., for a monocot or a dicot). Various plants of interest are disclosed elsewhere herein.

A seed coating can further comprise at least at least one nutrient, at least one herbicide or at least one pesticide, or at least one biocide. See, for example, US App Pub. 20040336049, 20140173979, and 20150033811.

In other embodiments, a plant of interest (i.e., plant susceptible to the plant disease) and/or the area of cultivation comprising the plant, can be treated with a combination of an effective amount of the bacterial strain and an effective amount of a biocide. By "treated with a combination of" or "applying a combination of" a bacterial strain and a biocide to a plant, area of cultivation or field it is intended that one or more of a particular field, plant, and/or weed is treated with an effective amount of the bacterial strain AIP61892 and one or more biocide so that a desired effect is achieved. Furthermore, the application of one or both of the bacterial strain and the biocide can occur prior to the planting of the crop (for example, to the soil, or the plant). Moreover, the application of the bacterial strain and the biocide may be simultaneous or the applications may be at different times (sequential), so long as the desired effect is achieved.

In one non-limiting embodiment, the (i.e., AIP61892, or a spore, or a forespore or a combination of cells, forespores and/or spores from AIP61892) is resistant to glyphosate. In such methods, a plant, crop, or area of cultivation is treated with a combination of an effective amount of the bacterial strain that is resistant to glyphosate and an effective amount of glyphosate, wherein the effective amount of glyphosate is such as to selectively control weeds while the crop is not significantly damaged.

In another non-limiting embodiment, the bacterial strain is resistant to glufosinate. In such methods, a plant, crop, or area of cultivation is treated with a combination of an effective amount of the bacterial strain that is resistant to glufosinate and an effective amount of glufosinate, wherein the effective amount of glufosinate is such as to selectively control weeds while the crop is not significantly damaged. In such embodiments, the effective amount of the bacterial strain is sufficient to result in a statistically significant increase in plant health, yield, and/or growth when compared to the plant health, yield, and/or growth that occurs when the same concentration of a bacterial strain that was not modified to be resistant to glufosinate is applied in combination with the effective amount of the glufosinate.

The bacterial strain comprises an effective amount of AIP61892, or a spore, or a forespore or a combination of cells, forespores and/or spores from AIP61892.

### V. Biocides for Use in Combination with the Bacterial Strain AIP61892.

As discussed elsewhere herein, the bacterial strain can be used in combination with a biocide (i.e., a herbicide, fungicide, pesticide, or other crop protection chemical). In such instances, the bacterial strain is compatible with the biocide of interest.

By "herbicide, fungicide, pesticide, or other crop protection chemical tolerance or herbicide, fungicide, pesticide, or other crop protection chemical resistance" is intended the ability of an organism (i.e., the plant and/or the bacterial strain) to survive and reproduce following exposure to a dose of the herbicide, fungicide, pesticide, or other crop protection chemical that is normally lethal to the wild type organism.

Herbicides that can be used in the various methods and compositions discloses herein include glyphosate, ACCase inhibitors (Arloxyphenoxy propionate (FOPS)); ALS inhibitors (Sulfonylurea (SU)), Imidazonlinone (IMI), Pyrimidines (PM)); microtubule protein inhibitor (Dinitroaniline (DNA)); synthetic auxins (Phenoxy (P)), Benzoic Acid (BA), Carboxylic acid (CA)); Photosystem II inhibitor (Triazine (TZ)), Triazinone (TN), Nitriles (NT), Benzothiadiazinones (BZ), Ureas (US)); EPSP Synthase inhibitor (glycines (GC)); Glutamine Synthesis inhibitor (Phosphinic Acid (PA)); DOXP synthase inhibitor (Isoxazolidinone (IA)); HPPD inhibitor (Pyrazole (PA)), Triketone (TE)); PPO inhibitors (Diphenylether (DE), N-phenylphthalimide (NP) (Ary triazinone (AT)); VLFA inhibitors (chloroacetamide (CA)), Oxyacetamide (OA)); Photosystem I inhibitor (Bipyridyliums (BP)); and the like.

Pesticides that can be used in the various methods and compositions disclosed herein include imidacloprid clothianidin, arylpyrazole compounds (WO2007103076); organophosphates, phenyl pyrazole, pyrethoids caramoyloximes, pyrazoles, amidines, halogenated hydrocarbons, carbamates and derivatives thereof, terbufos, chloropyrifos, fipronil, chlorethoxyfos, telfuthrin, carbofuran, imidacloprid, tebupirimfos (U.S. Patent No. 5,849,320).

Fungicides that can be used in the various methods and compositions disclosed herein include aliphatic nitrogen fungicides (butylamine, cymoxanil, dodicin, dodine, guazatine, iminoctadine); amide fungicides (benzovindiflupyr, carpropamid, chloraniformethan, cyflufenamid, diclocymet, diclocymet, dimoxystrobin, fenaminstrobin, fenoxanil, flumetover, furametpyr, isofetamid, isopyrazam, mandestrobin, mandipropamid, metominostrobin, orysastrobin, penthiopyrad, prochloraz, quinazamid, silthiofam, triforine); acylamino acid fungicides (benalaxyl, benalaxyl-M, furalaxyl, metalaxyl, metalaxyl-M, pefurazoate, valifenalate); anilide fungicides (benalaxyl, benalaxyl-M, bixafen, boscalid, carboxin, fenhexamid, fluxapyroxad, isotianil, metalaxyl, metalaxyl-M, metsulfovax, ofurace, oxadixyl, oxycarboxin, penflufen, pyracarbolid, sedaxane, thifluzamide, tiadinil, vanguard); benzanilide fungicides (benodanil, flutolanil, mebenil, mepronil, salicylanilide, tecloftalam); furanilide fungicides (fenfuram, furalaxyl, furcarbanil, methfuroxam); sulfonanilide fungicides (flusulfamide); benzamide fungicides (benzohydroxamic acid, fluopicolide, fluopyram, tioxymid, trichlamide, zarilamid, zoxamide); furamide fungicides (cyclafuramid, furmecyclox); phenylsulfamide fungicides (dichlofluanid, tolylfluanid); sulfonamide fungicides (amisulbrom, cyazofamid); valinamide fungicides (benthiavalicarb, iprovalicarb); antibiotic fungicides (aureofungin, blasticidin-S, cycloheximide, griseofulvin, kasugamycin, moroxydine, natamycin, polyoxins, polyoxorim, streptomycin, validamycin); strobilurin fungicides (fluoxastrobin, mandestrobin); methoxyacrylate strobilurin fungicides (azoxystrobin, bifujunzhi, coumoxystrobin, enoxastrobin, flufenoxystrobin, jiaxiangjunzhi, picoxystrobin, pyraoxystrobin); methoxycarbanilate strobilurin fungicides (pyraclostrobin, pyrametostrobin, triclopyricarb); methoxyiminoacetamide strobilurin fungicides (dimoxystrobin, fenaminstrobin, metominostrobin, orysastrobin); methoxyiminoacetate strobilurin fungicides (kresoxim-methyl, trifloxystrobin); aromatic fungicides (biphenyl, chlorodinitronaphthalenes, chloroneb, chlorothalonil, cresol, dicloran, fenjuntong, hexachlorobenzene, pentachlorophenol, quintozene, sodium pentachlorophenoxide, tecnazene, trichlorotrinitrobenzenes); arsenical fungicides (asomate, urbacide); aryl phenyl ketone fungicides (metrafenone, pyriofenone); benzimidazole fungicides (albendazole, benomyl, carbendazim, chlorfenazole, cypendazole, debacarb, fuberidazole, mecarbinzid, rabenzazole, thiabendazole); benzimidazole precursor fungicides (furophanate, thiophanate, thiophanate-methyl); benzothiazole fungicides (bentaluron, benthiavalicarb, benthiazole, chlobenthiazone, probenazole); botanical fungicides (allicin, berberine, carvacrol, carvone, osthol, sanguinarine, santonin); bridged diphenyl fungicides (bithionol, dichlorophen, diphenylamine, hexachlorophene, parinol); carbamate fungicides (benthiavalicarb, furophanate, iodocarb, iprovalicarb, picarbutrazox, propamocarb, pyribencarb, thiophanate, thiophanate-methyl, tolprocarb); benzimidazolylcarbamate fungicides (albendazole, benomyl, carbendazim, cypendazole, debacarb, mecarbinzid); carbanilate fungicides (diethofencarb, pyraclostrobin, pyrametostrobin, triclopyricarb); conazole fungicides, conazole fungicides (imidazoles) (climbazole, clotrimazole, imazalil, oxpoconazole, prochloraz, triflumizole); conazole fungicides (triazoles) (azaconazole, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, prothioconazole, quinconazole, simeconazole, tebuconazole, tetraconazole, triadimefon, triadimenol, triticonazole, uniconazole, uniconazole-P); copper fungicides (acypetacs-copper, Bordeaux mixture, Burgundy mixture, Cheshunt mixture, copper acetate, copper carbonate, basic, copper hydroxide, copper naphthenate, copper oleate, copper oxychloride, copper silicate, copper sulfate, copper sulfate, basic, copper zinc chromate, cufraneb, cuprobam, cuprous oxide, mancopper, oxine-copper, saisentong, thiodiazole-copper); cyanoacrylate fungicides (benzamacril, phenamacril); dicarboximide fungicides (famoxadone, fluoroimide); dichlorophenyl dicarboximide fungicides (chlozolinate, dichlozoline, iprodione, isovaledione, myclozolin, procymidone, vinclozolin); phthalimide fungicides (captafol, captan, ditalimfos, folpet, thiochlorfenphim); dinitrophenol fungicides (binapacryl, dinobuton, dinocap, dinocap-4, dinocap-6, meptyldinocap, dinocton, dinopenton, dinosulfon, dinoterbon, DNOC); dithiocarbamate fungicides (amobam, asomate, azithiram, carbamorph, cufraneb, cuprobam, disulfiram, ferbam, metam, nabam, tecoram, thiram, urbacide, ziram); cyclic dithiocarbamate fungicides (dazomet, etem, milneb); polymeric dithiocarbamate fungicides (mancopper, mancozeb, maneb, metiram, polycarbamate, propineb, zineb); dithiolane fungicides (isoprothiolane, saijunmao); fumigant fungicides (carbon disulfide, cyanogen, dithioether, methyl bromide, methyl iodide, sodium tetrathiocarbonate); hydrazide fungicides (benquinox, saijunmao); imidazole fungicides (cyazofamid, fenamidone, fenapanil, glyodin, iprodione, isovaledione, pefurazoate, triazoxide); conazole fungicides (imidazoles) (climbazole, clotrimazole, imazalil, oxpoconazole, prochloraz, triflumizole); inorganic fungicides (potassium azide, potassium thiocyanate, sodium azide, sulfur, see also copper fungicides, see also inorganic mercury fungicides); mercury fungicides; inorganic mercury fungicides (mercuric chloride, mercuric oxide, mercurous chloride); organomercury fungicides ((3-ethoxypropyl)mercury bromide, ethylmercury acetate, ethylmercury bromide, ethylmercury chloride, ethylmercury 2,3-dihydroxypropyl mercaptide, ethylmercury phosphate, N-(ethylmercury)-*p*-toluenesulphonanilide, hydrargaphen, 2-methoxyethylmercury chloride, methylmercury benzoate, methylmercury dicyandiamide, methylmercury pentachlorophenoxide, 8-phenylmercurioxyquinoline, phenylmercuriurea, phenylmercury acetate, phenylmercury chloride, phenylmercury derivative of pyrocatechol, phenylmercury nitrate, phenylmercury salicylate, thiomersal, tolylmercury acetate); morpholine fungicides (aldimorph, benzamorf, carbamorph, dimethomorph, dodemorph, fenpropimorph, flumorph, tridemorph); organophosphorus fungicides (ampropylfos, ditalimfos, EBP, edifenphos, fosetyl, hexylthiofos, inezin, iprobenfos, izopamfos, kejunlin, phosdiphen, pyrazophos, tolclofos-methyl, triamiphos); organotin fungicides (decafentin, fentin, tributyltin oxide); oxathiin fungicides (carboxin, oxycarboxin); oxazole fungicides (chlozolinate, dichlozoline, drazoxolon, famoxadone, hymexazol, metazoxolon, myclozolin, oxadixyl, oxathiapiprolin, pyrisoxazole, vinclozolin); polysulfide fungicides (barium polysulfide, calcium polysulfide, potassium polysulfide, sodium polysulfide); pyrazole fungicides (benzovindiflupyr, bixafen, fenpyrazamine, fluxapyroxad, furametpyr, isopyrazam, oxathiapiprolin, penflufen, penthiopyrad, pyraclostrobin, pyrametostrobin, pyraoxystrobin, rabenzazole, sedaxane); pyridine fungicides (boscalid, buthiobate, dipyrithione, fluazinam, fluopicolide, fluopyram, parinol, picarbutrazox, pyribencarb, pyridinitril, pyrifenox, pyrisoxazole, pyroxychlor, pyroxyfur, triclopyricarb); pyrimidine fungicides (bupirimate, diflumetorim, dimethirimol, ethirimol, fenarimol, ferimzone, nuarimol, triarimol); anilinopyrimidine fungicides (cyprodinil, mepanipyrim, pyrimethanil); pyrrole fungicides (dimetachlone, fenpiclonil, fludioxonil, fluoroimide); quaternary ammonium fungicides (berberine, sanguinarine); quinoline fungicides (ethoxyquin, halacrinate, 8-hydroxyquinoline sulfate, quinacetol, quinoxyfen, tebufloquin); quinone fungicides (chloranil, dichlone, dithianon); quinoxaline fungicides (chinomethionat, chlorquinox, thioquinox); thiadiazole fungicides (etridiazole, saisentong, thiodiazole-copper, zinc thiazole); thiazole fungicides (ethaboxam, isotianil, metsulfovax, octhilinone, oxathiapiprolin, thiabendazole, thifluzamide); thiazolidine fungicides (flutianil, thiadifluor); thiocarbamate fungicides (methasulfocarb, prothiocarb); thiophene fungicides (ethaboxam, isofetamid, silthiofam); triazine fungicides (anilazine); triazole fungicides (amisulbrom, bitertanol, fluotrimazole, triazbutil); conazole fungicides (triazoles) (azaconazole, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, huanjunzuo, imibenconazole, ipconazole, metconazole, myclobutanil, penconazole, propiconazole, prothioconazole, quinconazole, simeconazole, tebuconazole, tetraconazole, triadimefon, triadimenol, triticonazole, uniconazole, uniconazole-P); triazolopyrimidine fungicides (ametoctradin); urea fungicides (bentaluron, pencycuron, quinazamid); zinc fungicides (acypetacs-zinc, copper zinc chromate, cufraneb, mancozeb, metiram, polycarbamate, polyoxorim-zinc, propineb, zinc naphthenate, zinc thiazole, zinc trichlorophenoxide, zineb, ziram); unclassified fungicides (acibenzolar, acypetacs, allyl alcohol, benzalkonium chloride, bethoxazin, bromothalonil, chitosan, chloropicrin, DBCP, dehydroacetic acid, diclomezine, diethyl pyrocarbonate, ethylicin, fenaminosulf, fenitropan, fenpropidin, formaldehyde, furfural, hexachlorobutadiene, methyl isothiocyanate, nitrostyrene, nitrothal-isopropyl, OCH, pentachlorophenyl laurate, 2-phenylphenol, phthalide, piperalin, propamidine, proquinazid, pyroquilon, sodium orthophenylphenoxide, spiroxamine, sultropen, thicyofen, tricyclazole), or mefenoxam.

The following examples are offered by way of illustration and not by way of limitation.

### EXAMPLES

### Example 1:

### Materials and methods

**Plant material:** The susceptible soybean cultivar Williams 82 was used in strain evaluation using the detached-leaf technique (Twizeyimana and Hartman, 2010) and using whole plant in growth chambers. Briefly, soybean seeds were sown in 18-cell plastic inserts that were filled with soil-less mix (Sunshine Mix, LC1; Sun Gro Horticulture Inc., Bellevue, WA) and placed inside a flat. Cells were fertilized at planting with slow-release pellets (Osmocote 19-6-12; 2 pellets per cm²). Flats were maintained inside a growth chamber (Percival Scientific, Inc., Boone, IA) maintained at 70% relative humidity (RH) with a daily cycle of 14 h of light and 10 h of darkness at 24 and 20°C, respectively.

**Bacterial strains:** Bacterial strains were plated on Luria Bertani medium or in liquid culture, CHA medium which consists of, per L, NaCl (5g), tryptone (10g), nutrient broth (8g), CaCl₂ (0.14mM), MgCl₂·6H₂O (0.2mM), and MnCl₂·4H₂O (0.01 mM) and were purified to obtain single colonies. Single colonies were characterized morphologically or using molecular techniques.

***Phakopsora pachyrhizi* isolate:** The isolate FL07-1 was used in all inoculations. The isolate is a single spore isolate obtained from infected soybean leaves collected from Gadseden County, Florida in 2007.

### Strain evaluation:

*Evaluation on detached-leaf*: Briefly, leaf disks (3-cm diameter each) were sprayed with 120 µl of the bacterial strain (1 × 10⁸ spores/ml of water) of *P. pachyrhizi.* Leaf disks were inoculated with 120 µl spore suspension (1 × 10⁴ spores/ml of sterile distilled water) of *P. pachyrhizi* a day after application of the bacterial strain. Both the bacterial strain and *P*. *pachyrhizi* inoculation applications were done using an atomizer attached to an air compressor (Twizeyimana and Hartman, 2010). Leaf disks were placed adaxial side down on saturated 20 × 20 cm filter paper (Whatman International Ltd., Kent, England) in a plastic container (Blister Box 20 × 20 cm, Placon, Madison, WI); two filter papers were used per box. Boxes with leaf disks were incubated in the dark for a period of 12 h followed by a cycle of 13 hours of light (380 umol m-2s-1) and 11 h of darkness inside a tissue chamber (Percival Scientific, Inc.) maintained at 23°C and 95% RH. Prior to incubation, boxes were placed inside zip bags (Webster Industries, Peabody, MA). The experimental design was a randomized complete block design with 2 replications and was repeated once.

***Evaluation on whole plant*:** Bacterial strains were selected from the initial screening (conducted using leaf disk) based on their uredinia counts and were evaluated on whole plant in growth chambers. In this evaluation, when plants were at V2-stage (Fehr et al. 1971), the first fully expanded trifoliate leaf was sprayed with the bacterial strain, and the inoculation with *P. pachyrhizi* was done a day after as described in detached-leaf evaluation. Sprayed plants were maintained in a growth chamber at 75% RH with a daily cycle of 14 h and 10 h of light and darkness at 22°C and 24°C, respectively. The experimental design was a randomized complete block design with 3 replications and was repeated once.

### Data collection and results:

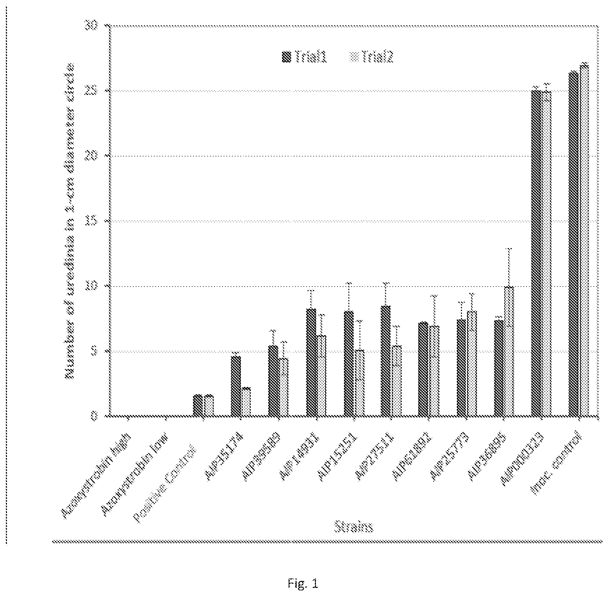

In both evaluations, data recorded were numbers of uredinia (uredinia counts) in 1-cm diameter circle recorded 14 days after inoculation. Nine bacterial strains that had < 10 uredinia in 1-cm diameter circle were selected after evaluation on whole plant in growth chamber to be tested in the field (Fig. 1 & Table 2).

**Table 2. Nine bacterial strains selected from evaluation on whole plant in growth chambers (bacterial strains other than AIP61892 do not form part of the invention)**

| # | **AIP** | **Strains** |
|---|---|---|
| 1 | 14931 | *Bacillus thuringiensis* |
| 2 | 15251 | *Bacillus frigoritolerans* |
| 3 | 25773 | *Bacillus flexus* |
| 4 | 27511 | *Bacillus drentensis* |
| 5 | 35174 | *Bacillus cereus* |
| 6 | 36895 | *Bacillus simplex* |
| 7 | 39589 | *Bacillus acidiceler* |
| 8 | 61892 | *Bacillus subtilis* subsp. *Subtilis* |
| 9 | 79428 | *Burkholderia vietnamiensis* |

### Example 2. Methods of Culturing (strains other than AIP61892 do not form part of the invention)

Bacterial strains were cultured in CHA media which consists of, per L, NaCl (5g), tryptone (10g), nutrient broth (8g), CaCl₂ (0.14mM), MgCl₂·6H₂O (0.2mM), and MnCl₂·4H₂O (0.01 mM). Table 3 summarizes the incubation time, the concentration of bacteria (CFU/ml) achieved and percentage of sporulation.

**Table 3**

| Strain | Medium | Incubation time (hrs) | Concentration (CFU/ml) | Sporulation |
|---|---|---|---|---|
| AIP23364 | CHA | 40 | 5e9 | Not tested |
| AIP 27511 | CHA | 50 | 1.25e9 | 50% |
| AIP 35174 | CHA | 50 | 1e9 | 80% |
| AIP 25773 | CHA | 50 | 8.3e8 | 100% |
| AIP 15251 | CHA | 50 | 8.4e8 | Did not sporulate |
| AIP 61892 | CHA | 50 | 1.3e9 | 90% |
| AIP 79428 | CHA | 50 | 5e9 | Did not sporulate |
| AIP 14931 | CHA | 50 | 2e8 | 50% |
| AIP 39589 | CHA | 46.5 | 8.7e8 | ²100% intermediate |
| AIP 36895 | CHA | 48 | 6.2e8 | Did not |
| | | | | sporulate |

| | | | | |
|---|---|---|---|---|
| ² Formation of forespore, did not form endospore | | | | |

### Example 3. Field Trials for the Various Bacterial Strains or Active Variants Thereof (Treatments not including AIP61892 are reference examples).

The various bacterial strains recited in Table 2 are applied to soybeans in the field. Treatments are applied at 157.15 Liters/Hectare (16.8 Gallons/Acre) with treatments applied to achieve uniform plant coverage per general treatment guidelines for ASR treatment. The first treatment is applied at R1 with a follow up treatment applied at 14 days and 28 days after first treatment. The specific treatments are outlined below.

Treatments:
1. Untreated Check
2. Inoculated Check
3. Quadris at 434.33 g/ha (6.2oz/acre)
4. Quadris at 147.11 g/ha (2.1oz/acre)
5. AIP27511 at 7.5g/L
6. AIP35174 at 7.5g/L
7. AIP25773 at 7.5g/L
8. AIP15251at 7.5g/L
9. AIP61892at 7.5g/L
10. AIP79428 at 7.5 g/L
11. AIP14931 at 7.5 g/L
12. AIP39589 at 7.5 g/L
13. AIP36895 at 7.5 g/L

### Example 4. Field Trials for the Various Bacterial Strains or Active Variants Thereof (Treatments not including AIP61892 are reference examples).

The various bacterial strains recited in Table 2 are applied to soybeans in the field. Bacterial strain treatments are applied at 187.08 Liters/Hectare (20 Gallons/Acre) with treatments applied to achieve uniform plant coverage per general treatment guidelines for ASR treatment. The first treatment is applied at R1 with a follow up treatment applied at 14 days after first treatment. The specific treatments are outlined below.

Treatments:
1. Untreated Check
2. Inoculated Check
3. Quadris at 434.33 g/ha (6.2oz/acre)
4. Quadris at 147.11 g/ha (2.1oz/acre)
5. AIP27511 at 7.5g/L
6. AIP35174 at 7.5g/L
7. AIP25773 at 7.5g/L
8. AIP15251at 7.5g/L
9. AIP61892at 7.5g/L
10. AIP79428 at 7.5 g/L
11. AIP14931 at 7.5 g/L
12. AIP39589 at 7.5 g/L
13. AIP36895 at 7.5 g/L

### Example 5. Rhizoctonia Damping-Off Assay On Soybean Mock Seed Treatment/In-Furrow

11-14 day old *Rhizoctonia solani* infested grain was ground. The ground inoculum was screed through a #10 screen to remove any grain that was not ground well. The ground, screened infected grains was added to Fafard Superfine Germination media at 1.5 grams of ground inoculum to 1 liter of soil mix by volume. Germination mix, inoculum, and 1 liter of water per 75 liters of germination media was added to a cement mixer and mixed until everything was well incorporated. The well incorporated media-inoculum material was placed into a secondary holding container with a lid and held at 20°C for 18 hours before using in the assay.

606-cell planting trays were filled with inoculated germination media making sure to not pack the media too firmly. One soybean seed was sown per 606 cell, planting at a depth of 1.5 to 2 cm leaving the planting holes open if applying treatments as a liquid formulation. Individual planting cells were treated with one of the re-suspended strains set forth in Table 1 at 3 ml per cell/seed. The seed treatment was directly over the top of the seed. Once treatments were applied, the shake flats were shaken lightly to close planting holes. The planting trays were lightly watered and placed in a humidity dome on the flat. After 3-4 days flats were checked for moisture and lightly watered as needed to ensure cells were evenly moist. The humidity dome was replaced after watering.

*Data Collection and Results:* After 10-12 days, the assay was evaluated to determine the number of seeds that germinated. Data are reported as the % of seeds that germinated out of a total of 6 seeds per treatment. Eight strains with germination rates ≥50% and comparable to the non-inoculated control were selected for field testing (Table 4).

**Table 4. Bacterial strains with activity against R. solani in the soybean seed germination assay.**

| (Treatments not including AIP61892 are reference examples). | | |
|---|---|---|
| **AIP** | **% germinated** | **Number of Reps** |
| AIP061892 | 64 | 8 |
| AIP079428 | 58 | 7 |
| Non-inoculated | 86 | 22 |
| Inoculated | 27 | 22 |

### Example 6. Methods of Formulation for the Various Bacterial Strains

The culture produced in Example 2 was centrifuged, 20 minutes, 10,000 rpm to produce a pellet. The supernatant was poured off and another volume of culture fluid was added to the previous pellet and centrifuged again (this was to reduce the number of centrifuge tubes required for each harvest).

End product material was produced by adding 5% (by mass of pellet) of glycerol to the cell pellet and then mixed with a spatula. 20% (by mass) of Microcel E was transferred to a food processor and the glycerol/pellet was poured over the microcell. This material was blended using the knife blade attachment of the food processor for not more than 10 seconds. The product was dried overnight at 40°C to approximately an a_{w} of 0.3.

### Example 7. Greenhouse Evaluation of Bacterial Strains Against Asian Soybean Rust

**Plant material and bacterial strains:** Cultivar Williams 82 was used in these experiments. The bacterial strains were prepared as follows. Fermentation culture broth was spun down and the pellet mass was weighed. For each 100 g of pellet material, 5 g of glycerol was added (5% of the pellet mass). Glycerol was mixed by hand until a uniform consistency is achieved. A total of 20 g (20% by weight of cell paste) of microcell-E (Imery's Celite) was added to a food processor equipped with a Sabatier blade. Cell paste, glycerol, and micro-cel were homogenized briefly into a partially dry, crumb-like structure. This end product was spread into aluminum trays and dried at 40°C, overnight. Once the product dryness reached a water activity of 0.3 or less, it was milled and screened and was stored at 4°C.

***Phakopsora pachyrhizi* isolate:** The isolate FL07-1 described above was used in experiments in Illinois and mixtures of spores collected in 2014 and 2015 were used in experiments in Florida.

**Greenhouse strain evaluation:** In Illinois, two trials were conducted in a biosafety level 2 greenhouse. The conditions in the greenhouse were set for 22±2°C under a 16-h photoperiod with supplemental illumination provided by 1,000-W Metalarc high-intensity lamps (Sylvania, Danvers, MA). Seeds of Williams 82 were sown in soil-less mix (Sunshine Mix, LC1; Sun Gro Horticulture Inc.) in 5-inch pots, and fertilized at planting with slow-release pellets (Osmocote 19-6-12; 1 to 2 pellets per cm²). Plants were thinned to one plant per pot after emergences and the experimental design was a randomized complete block design with 4 replicates for each treatment. Plants were sprayed with strains according to the protocol at growth stage V2-stage (Fehr et al. 1971), and they were inoculated with a spore suspension of isolate FL07-1 (1 × 10⁵ spores/ml of sterile distilled water) until runoff using a hand sprayer a day after strain application. Inoculated plants were left in mist chamber overnight and then were moved to greenhouse bench for symptom development. Fourteen days later, plants were retreated according to protocol. Six days after the second treatment, rust severity data was recorded.

In Florida, seeds of Williams 82 were sown into 22.8-cm-diameter plastic pots containing Metro Mix 300 (Sun GroHorticultural Distributors Inc., Bellevue, WA). Plants were maintained in a rust-free glass green-house on metal benches at an average temperature of 26°C and an average relative humidity of 61%. Plants were thinned to one plant per pot after emergences. The experimental design was a randomized complete block design with 3 replicates for each treatment. Plants were sprayed with strains according to the protocol at growth stage R1-stage (Fehr et al. 1971), and were inoculated a day later using a mixture of *P. pachyrhizi.* Strain treatments were reapplied 14 days after the first treatments. Rust severity was recorded when plants were at R4- or 5-stage.

**Data Collection:** In Illinois, rust severity was scored by counting the number of sporulating uredinia in an arbitrarily selected 1-cm diameter circle from each leaflet of inoculated trifoliate leaves. The data is shown in Table 5. In Florida, the data recorded were percent soybean rust severity from a randomly selected plant. The data is shown in Table 6. In both tables, treatments not including AIP61892 are reference examples.

**Table 5. Number of sporulating uredinia per 1-cm diameter circle of leaf tissue treated with different bacterial strains in the greenhouse experiment conducted in Illinois. A (-) indicates "not tested". Samples represented by a different letter (eg., a, ab, b, c, cd, d, e) had statistically significant different values.**

| Strain | Number of sporulating uredinia per 1-cm dia circle | |
|---|---|---|
| | Trial 1 | Trial 2 |
| AIP039589 | 4.2 b | 3.5 ab |
| AIP027511 | - | - |
| AIP035174 | 3.0 ab | 17.8 d |
| AIP025773 | 10.3 | - |
| AIP015251 | 7.0 c | 15.2 d |
| AIP061892 | 5.1 b | 3.6 ab |
| AIP079428 | - | 4.2 b |
| AIP014931 | 7.8 c | 24.1 e |
| AIP036895 | - | - |
| Fungicide (Quadris) | 0 a | 0 a |
| Inoculated Control | 12.7 d | 28.5 e |

**Table 6. Percent rust severity of soybean plants treated with different bacterial strains in the greenhouse experiment conducted in Florida. A (-) indicates "not tested". Samples represented by a different letter (eg., a, ab, b, c, cd, d, e) had statistically significant different values.**

| Strain | % Average rust severity |
|---|---|
| AIP039589 | 13.3 b |
| AIP027511 | - |
| AIP035174 | 17.1 bc |
| AIP025773 | 15.9 bc |
| AIP015251 | 21.0 d |
| AIP061892 | 11.0 b |
| AIP079428 | - |
| AIP014931 | 23.8 d |
| AIP036895 | - |
| Fungicide (Quadris) | 1.3 a |
| Inoculated Control | 22.4 d |

### Example 8. Field Evaluations Against Asian Soybean Rust in Florida

A field experiment was conducted in Florida in 2015 at the North Florida Research and Education Center in Quincy. The average monthly temperature from July to September during evaluation, ranged from 24 to 27°C. The plant material, bacterial strains and *P*. *pachyrhizi* isolate were similar to those described in the greenhouse experiment in Florida (Example 7). Plants were sprayed with strains according to the protocol at R1 growth stage (Fehr *et al.* 1971) and were inoculated a day after using a mixture of *P. pachyrhizi* that was followed by subsequent natural infection. Strain treatments were reapplied at 14 days after the first treatment. Percent soybean rust severity was recorded from a randomly selected plant at R5-stage. The data is shown in Table 7.

**Table 7. Percent rust severity of soybean plants treated with different bacterial strains in the field experiment conducted in Florida A (-) indicates "not tested" Samples resented by a different letter (eg., a, ab) had statistically significant different values.**

| (Treatments not including AIP61892 are reference examples). | |
|---|---|
| Strains | % Average rust severity |
| AIP039589 | - |
| AIP027511 | - |
| AIP035174 | 6.4 ab |
| AIP025773 | 3.4 a |
| AIP015251 | 3.9 a |
| AIP061892 | 3.6 a |
| AIP079428 | - |
| AIP014931 | 2.9 a |
| AIP036895 | - |
| Fungicide (Quadris) | 2.5 a |
| Inoculated Control | 5.8 ab |

### Example 9. Rhizoctonia Damping-Off Assay-- Soybean Mock Seed Treatment/In-Furrow

11-14 day old *Rhizoctonia solani* infested grain is ground. The ground inoculum is screened through a #10 screen to remove any grain that is not ground well. The ground, screened infected grains are added to Fafard Superfine Germination media at 1.5 grams of ground inoculum to 1 liter of soil mix by volume. Germination mix, inoculum, and 1 liter of water per 75 liters of germination media are added to a cement mixer and mix until everything is well incorporated. The well incorporated media-inoculum material is placed into a secondary holding container with a lid and held at 20°C for 18 hours before using in the assay.

606-cell planting trays are filled with inoculated germination media making sure to not pack the media too firmly. One soybean seed is sown per 606 cell, planting at a depth of 1.5 to 2cm leaving the planting holes open if applying treatments as a liquid formulation. Individual planting cells are treated with one of the re-suspended strains set forth in Table 2 at 3ml per cell/seed. The seed treatment is directly over the top of the seed. Once treatments is applied, the shake flats is shaken lightly shake to close planting holes. The planting trays are lightly watered and placed in a humidity dome on the flat. After 3-4 days, flats are checked for moisture and lightly watered as needed to ensure cells are evenly moist. The humidity dome is replaced after watering.

*Data Collection and Results*: After 10-12 days, the assay is evaluated to determine the number of seeds that germinated. Data is reported as the % of seeds that germinated out of a total of 6 seeds per treatment.

### Example 10. Field Trials Against Various Fungal Pathogens for the Various Bacterial Strains (Treatments not including AIP61892 are reference examples).

The various bacterial strains recited in Table 2 are applied to the crops listed in Table 8 in the field under the current agronomic practices at listed in Table 8 to achieve uniform plant coverage and follow proper agronomic practices. Treatments are applied preventatively and/or curatively at the appropriate timings per disease.

**Table 8**

| **Crop** | **Pathogen** | **Rate** | **Treatment Volume** | **Treatment Number** | **Application Interval/Timing** |
|---|---|---|---|---|---|
| All crops | Gray Mold | 5g/L | 233.85 - 1870.79 L/ha (25-200 Gallons/Acre) | 1 to 10 | 7 to 14 days |
| Ornamental Crops | Cercospora Leaf Spots | 5g/L | 935.40 - 2806.19 L/ha (100-300 Gallons/Acre) | 1 to 4 | 7 to 14 days |
| Soybean | Cercospora Leaf Spots | 5g/L | 46.77 - 187.08 L/ha (5-20 Gallons/Acre) | 1 to 3 | V7, R1, R3, R5 |
| Beet, Spinach, Chard | Cercospora Leaf Spots | 5g/L | 140.31 - 467.70 L/ha (15-50 Gallons/Acre) | 3 to 6 | 7 to 14 days |
| Solanaceous Crops | Early Blight | 5g/L | 140.31 - 467.70 L/ha (15-50 Gallons/Acre) | 4 to 10 | 7 to 14 days |
| Grape | Powdery Mildew | 5g/L | 140.31 - 467.70 L/ha (15-50 Gallons/Acre) | 3 to 8 | 7 to 14 days |
| Cucurbit | Powdery Mildew | 5g/L | | 2 to 8 | 7 to 14 days |
| Turf/other grasses | Anthrancose leaf spot | 5g/L | 813.79 - 1122.47 L/ha (87-120 Gallons/Acre) | 2 to 6 | 7 to 14 days |
| Grape | Downy Mildew | 5g/L | 467.70 - 935.40 L/ha (50-100 Gallons/Acre) | 2 to 6 | 7 to 14 days |
| Leafy Greens | Downy Mildew | 5g/L | 233.85 - 701.55 L/ha (25 to 75 Gallons/Acre) | 2 to 6 | 7 to 14 days |
| Basil | Downy Mildew | 5g/L | 233.85 - 701.55 L/ha (25-75 Gallons/Acre) | 2 to 6 | 7 to 14 days |
| Ornamental Plants | Late Blight | 5g/L | 935.40 - 2806.19 L/ha (100-300 Gallons/Acre) | 2 to 6 | 7 to 14 days |
| Cucurbit/Peppers | Late Blight | 5g/L | 233.85 - 935.40 L/ha (25-100 Gallons/Acre) | 2 to 10 | 7 to 14 days |
| Solanaceous Crops | Late Blight | 5g/L | 233.85 - 935.40 L/ha (25-100 Gallons/Acre) | 2 to 10 | 7 to 14 days |
| Soybean | Late Blight | 5g/L | 46.77 - 187.08 L/ha (5-20 Gallons/Acre) | 1 to 3 | V4 to R5 |
| Soybean | Rust | 5g/L | 46.77 - 187.08 L/ha (5-20 Gallons/Acre) | 1 to 4 | V4 to R5 |
| Rosacea family | Fire Blight | 5g/L | 187.08 - 935.40 L/ha (20-100 Gallons/Acre) | 1 to 3 | Pre/Post Flower |
| Malus | Apple Scab | 5g/L | 187.08 - 935.40 L/ha (20-100 Gallons/Acre) | 1 to 5 | 7 to 14 days |
| Stone Fruits | Brown Rot | 5g/L | 187.08 - 935.40 L/ha (20-100 Gallons/Acre) | 1 to 3 | Pre/Post Flower and Fruit Set |
| Rice | Sheath Blight | 5g/L | 46.77 - 187.08 L/ha (5-20 Gallons/Acre) | 1 to 3 | Prior to Canopy Closure |
| Cereals | Fusarium Head Blight | 5g/L | 46.77 - 187.08 L/ha (5-20 Gallons/Acre) | 1 to 2 | Feekes 7, 9, and/or 10.51 |

The specific treatments are outlined below:
Foliar Pathogen Treatment List: Early Blight
6-10 treatments
Treatment Volume: 935.40 L/ha (100 gallons/acre)
Treatment List:
   1. Non-Inoculated, untreated Check
   2. Inoculated Check
   3. Chemical control chosen by cooperator applied at label instructions
   4. Biological control Serenade applied at label instructions
   5. Experimental Biological Foliar treatment(s) at 5g/L plus Capsil at 0.2247 g/L (3 oz/100 gallons)

### Example 11. Field Trials Against Various Fungal Pathogens for the Various Bacterial Strains or active Variants Thereof employing Seed Treatments (Treatments not including AIP61892 are reference examples).

The various bacterial strains recited in Table 2 are applied to the crops listed in Table 9 as seed treatments prior to being planted into the field. Bacterial strain treatments are applied for preventative control of the diseases and at the application rates in Table 10. The specific treatments are outlined below.

**Table 9**

| | | | |
|---|---|---|---|
| Soybean | Canola | Wheat | Cereal Grains |
| Maize | Cucurbit | Cotton | Solanaceous Crops |
| Beets | Leafy Greens | Verticillium Whilt | Sunflower oil and seed |

Seed Treatment Trial Treatment List:
1. Non-inoculated Check
2. Inoculated Check
3. Disease appropriate Seed Treatment Chemical Check chosen and applied by cooperator
5. Biological Experimental Seed Treatment(s)

**Table 10**

| **Crop** | **Pathogen** | **Rate** | **Treatment Type** |
|---|---|---|---|
| Row Crops/Vegetables | Pythium | 10e4 to 10e12 | Seed Treatment |
| Row Crops/Vegetables | Phytophthora | 10e4 to 10e12 | Seed Treatment |
| Row Crops/Vegetables | Fusarium Wilt | 10e4 to 10e12 | Seed Treatment |
| Row Crops/Vegetables | Soybean Death Syndrome | 10e4 to 10e12 | Seed Treatment |
| Row Crops/Vegetables | Rhizoctonia solani | 10e4 to 10e12 | Seed Treatment |
| Row Crops/Vegetables | Verticillium Wilt | 10e4 to 10e12 | Seed Treatment |
| Row Crops/Vegetables | Corn Stalk Rot | 10e4 to 10e12 | Seed Treatment |

### Example 12. Field Trials Against Various Fungal Pathogens for the Various Bacterial

### Strains or Active Variants Thereof Employing In-Furrow Treatments (Treatments not including AIP61892 are reference examples).

The various bacterial strains or active variants thereof recited in Table 1 are applied to the crops listed in Table 9 as in-furrow treatments at time of planting as preventative control for the diseases and at the treatment rates listed in Table 11. The specific treatments are outlined below:
In-Furrow Trial Treatment List:
1. Non-inoculated Check
2. Inoculated Check
3. In-Furrow Biological Treatment(s) 5g/L + Capsil at 0.4493 g/L (6oz/100 Gallons) at 140.31 L/ha (15 Gallons/Acre)
4. Disease appropriate In-Furrow Chemical Check as chosen and applied by cooperator.

**Table 11**

| **Crop** | **Pathogen** | **Rate** | **Treatment/Volume** |
|---|---|---|---|
| Row Crops/Vegetables | Pythium | 5g/L | 18.71 - 140.31 L/ha (2 to 15 Gallons/Acre) |
| Row Crops/Vegetables | Phytophthora | 5g/L | 18.71 - 140.31 L/ha (2 to 15 Gallons/Acre) |
| Row Crops/Vegetables | Fusarium Wilt | 5g/L | 18.71 - 140.31 L/ha (2 to 15 Gallons/Acre) |
| Row Crops/Vegetables | Soybean Death Syndrome | 5g/L | 18.71 - 140.31 L/ha (2 to 15 Gallons/Acre) |
| Row Crops/Vegetables | Rhizoctonia solani | 5g/L | 18.71 - 140.31 L/ha (2 to 15 Gallons/Acre) |
| Row Crops/Vegetables | Verticillium Wilt | 5g/L | 18.71 - 140.31 L/ha (2 to 15 Gallons/Acre) |
| Row Crops/Vegetables | Corn Stalk Rot | 5g/L | 18.71 - 140.31 L/ha (2 to 15 Gallons/Acre) |

### Example 13- Biological Control Strain Seed Treatment Protocol

The seed treatment formulation was made by mixing 10g formulated strain plus 30ml water plus 15ml Unicoat Polymer. The weighed out seed is placed in a sterilized mason jar. An appropriate amount of seed treatment solution based off of seed weight (.05ml/25g seed), the mixture is shaken for 60 seconds or until the seeds were visually well coated. The seeds are placed into a single layer in a foil roasting pan and placed under a laminar flow hood for 1 hour or until seeds are dry. Once the seeds dry, they are placed in an air tight container and stored at RT.

### Example 14. Wettable Powder Formulations (Formulations not including AIP61892 are not part of the invention).

One hundred grams of cell paste from each of the strains denoted below in Table 12 was mixed with 5 g of glycerol and 20 g of synthetic calcium silicate using a food processor. This material was dried at 40°C to a water activity of less than 0.30 at which time it contained CFU/g as noted in Table 12. The dried powder formulation was stored in vacuum sealed mylar pouches at 22 C. The dried powder formulation retained antifungal activity.

**Table 12**

| Strain Name | CFU/g wettable powder |
|---|---|
| AIP14931 | 1.16x10¹¹ |
| AIP15251 | 1.47x10¹¹ |
| AIP25773 | 6.90x10¹⁰ |
| AIP35174 | 123x10¹¹ |
| AIP61892 | 6x10¹⁰ |
| AIP79428 | 4.73x10⁹ |

### Example 15. Pythium Field Trials.

The bacterial strains AIP061892 and AIP079428 (reference example) were applied as seed treatments to Soybean variety W3103. The bacterial strains were each formulated as a wettable powder as described in Example 14 and then turned into seed treatments by combining 10g of formulated bacterial strain with 30ml water and 15ml Seed Coating Polymer (Unicoat) and then shaking until a uniform solution was made. The finished solution was applied to 1kg of soybean seed and allowed to dry under a laminar flow hood for 12 hours
*Pythium* inoculum was grown on millet grain and applied via in-furrow application at 4.101 g/m (1.25g/ft) and was applied at planting with treated soybeans seeded at 52,650 seeds per ha (130,000 seeds per acre) on day 1. Whole row stand counts were taken 17 days later. The specific treatments are outlined below.

Treatments:
1. Untreated Check
2. Inoculated Check
3. Quadris at 0.029 L/ha (0.4 fluid ounces/Acre)
4. AIP061892 Seed Treatment
5. AIP079428 Seed Treatment

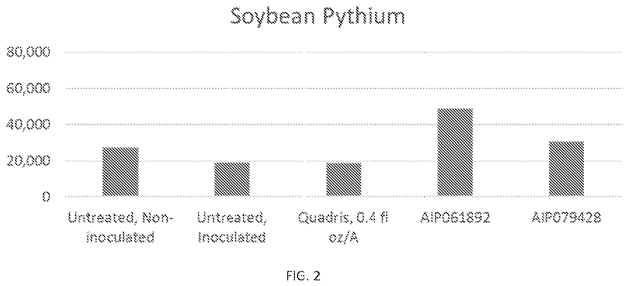

Fig. 2 shows the number of germinated seedlings (stand count) per Hectar. Fig. 2 demonstrates that AIP061892 and AIP079428 each produced about a 2-fold increase in germination over inoculated control.

### Example 16. Rhizoctonia solani Field Trials.

The bacterial strains AIP061892 and AIP079428 (reference example) were applied as seed treatments to Soybean variety W3103. The bacterial strains were each formulated as a wettable powder as noted in Example 14 and then turned into seed treatments by combining 10g of formulated bacterial strain with 30ml water and 15ml Seed Coating Polymer (Unicoat) and then shaking until a uniform solution was made. The finished solution was applied to 1kg of soybean seed and allowed to dry under a laminar flow hood for 12 hours.

*Rhizoctonia solani* inoculum was grown on sorghum grain and applied via in-furrow application at 4.101 g/m (1.25g/ft) and was applied at planting with treated soybeans seeded at 52,650 seeds per ha (130,000 seeds per acre) on day 1. Whole row stand counts were taken 17 days later. The specific treatments are outlined below:
Treatments:
1. Untreated Check
2. Inoculated Check
3. Quadris at 0.029 L/ha (0.4 fluid ounces/Acre)
4. AIP061892 Seed Treatment
5. AIP079428 Seed Treatment

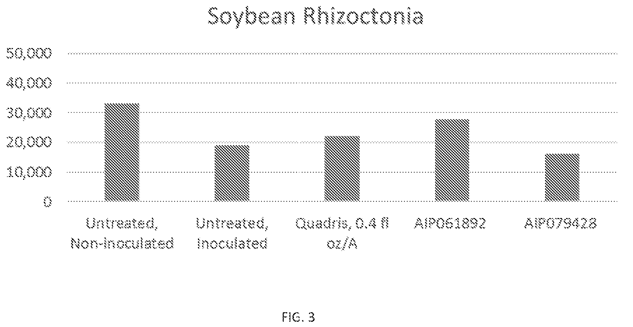

Fig. 3 shows the number of germinated seedlings (stand count) per Hectar. Fig. 3 demonstrates that AIP061892 produced a 50% recovery in germination over inoculated control.

## Claims

1. A composition comprising:
(a) bacterial strain AIP61892; and/or
(b) at least one of a spore, or a forespore, or a combination of cells, forespores and/or spores from AIP61892;
wherein said bacterial strain, spore, or a forespore, or a combination of cells, forespores and/or spores is present at 10⁵ CFU/gram to 10¹² CFU/gram or at 10⁵ CFU/ml to 10¹² CFU/ml, and wherein an effective amount of said composition improves an agronomic trait of interest of the plant or controls a fungal plant pathogen that causes a fungal plant disease.

2. The composition of claim 1, wherein said composition comprises (a) a cell paste, or (b) a wettable powder, or (c) a spray dried formulation, or (d) a stable formulation.

3. An isolated biologically pure culture of a bacterial strain comprising:
(a) AIP61892; or,
(b) a spore, or a forespore, or a combination of cells, forespores and/or spores from AIP61892,preferably,
wherein said bacterial strain is resistant to a biocide selected from a herbicide, a fungicide, a pesticide, or a crop protection chemical, wherein said culture is produced by growing in the presence of said biocide, wherein said bacterial strain controls a fungal plant pathogen that causes a fungal plant disease.

4. The isolated biologically pure culture of claim 3, wherein said biologically pure culture is able to grow in the presence of glyphosate.

5. A bacterial culture comprising
(a) AIP61892; or,
(b) a spore, or a forespore, or a combination of cells, forespores and/or spores from AIP61892;
wherein said bacterial culture has antipathogenic activity against a fungal plant pathogen that causes a fungal plant disease and is able to grow in the presence of glufosinate or an effective amount of said bacterial culture improves an agronomic trait of interest of the plant.

6. A method for growing a plant susceptible to a fungal plant disease or improving a agronomic trait of interest in a plant comprising applying to the plant
(a) an effective amount of bacterial strain AIP61892; and/or,
(b) an effective amount of at least one of a spore, or a forespore, or a combination of cells, forespores and/or spores from AIP61892;
wherein said effective amount comprises at least 10¹² to 10¹⁶ colony forming units (CFU) per hectare, and wherein said effective amount controls a fungal plant pathogen that causes the fungal plant disease or improves the agronomic trait of interest.

7. The method of claim 6, wherein said method increases yield of the plant susceptible to the fungal plant disease.

8. The method of claim 6 or 7, wherein the fungal plant disease is Asian Soybean Rust (ASR) or damping off complex.

9. The method of any one of claims 6-8, wherein the fungal plant pathogen comprises one or more fungal pathogens selected from the group consisting of *Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaequalis, Verticillium spp, Monilinia fructicola, Monilinia lax, and Monilinia fructigena,* more preferably,
wherein said fungal plant pathogen comprises one or more fungal pathogens selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi, and Phakopsora meibomiae.*

10. A method of controlling a fungal plant pathogen that causes a fungal plant disease in an area of cultivation comprising:
(a) planting the area of cultivation with seeds or plants susceptible to the fungal plant disease; and
(b) applying to the plant susceptible to the fungal plant disease an effective amount of at least one bacterial strain comprising
i. AIP61892; or,
ii. a spore, or a forespore, or a combination of cells, forespores and/or spores from AIP61892,
wherein said effective amount comprises at least 10⁵ to 10¹⁶ colony forming units (CFU) per hectare.

11. The method of claim 10, wherein said plant is susceptible to Asian Soybean Rust (ASR) or damping off complex.

12. The method of claim 10 or 11, wherein the fungal plant pathogen comprises one or more fungal pathogens selected from the group consisting of *Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp.,Venturia inaequalis, Verticillium spp, Monilinia fructicola, Monilinia lax, and Monilinia fructigena,* more preferably,
wherein the fungal plant pathogen comprises one or more fungal pathogens selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi, and Phakopsora meibomiae.*

13. The method of any one of claim 10-12, wherein said method further comprises applying an effective amount of a biocide, wherein said effective amount of the biocide selectively controls an organism of interest while not significantly damaging the crop, preferably,
wherein the bacterial strain and the biocide are applied simultaneously, or,
wherein the bacterial strain and the biocide are applied sequentially.

14. The method of claim 13, wherein the biocide is a fungicide.

15. A method of making a modified bacterial strain having increased resistance to a biocide of interest, the method comprising:
(a) providing a population of a bacterial strain comprising AIP61892, wherein said bacterial strain is susceptible to the biocide of interest;
(b) culturing said bacterial strain in the presence of the biocide of interest; and,
(c) selecting a modified bacterial strain having an increased resistance to said biocide of interest, wherein the modified bacterial strain retains the ability to control one or more fungal plant diseases and/or control one or more fungal plant pathogens.

16. The method of claim 15, wherein said culturing comprises increasing the concentration of the biocide over time.

17. The method of claim 15 or 16, wherein said biocide is glyphosate or glufosinate.

18. A method of treating or preventing a fungal plant disease comprising applying to a plant having a fungal plant disease or at risk of developing a fungal plant disease an effective amount of a bacterial strain comprising:
(a) AIP61892; and/or
(b) at least one of a spore or a forespore, or a combination of cells, forespores and/or spores from AIP61892,
wherein said effective amount comprises at least 10¹² to 10¹⁶ CFU per hectare, and wherein said bacterial strain controls a fungal plant pathogen that causes the fungal plant disease.

19. The method of claim 18, wherein the bacterial strain treats or prevents one or more fungal plant diseases, preferably, wherein the one or more fungal plant diseases comprise Asian Soybean Rust (ASR) or damping off complex.

20. The method of claim 18 or 19, wherein the bacterial strain controls one or more fungal plant pathogens.

21. The method of claim 20, wherein the one or more fungal plant pathogens are selected from the group consisting of *Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp.,Venturia inaequalis, Verticillium spp, Monilinia fructicola, Monilinia lax, and Monilinia fructigena,* more preferably,
wherein the one or more fungal pathogens are selected from the group consisting of *Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi, and Phakopsora meibomiae.*

## Patentansprüche

1. Zusammensetzung, umfassend:
(a) den Bakterienstamm AIP61892 und/oder
(b) eine Spore und/oder eine Vorspore und/oder eine Kombination von Zellen, Vorsporen und/oder Sporen von AIP61892,
wobei der Bakterienstamm, die Spore oder eine Vorspore oder eine Kombination von Zellen, Vorsporen und/oder Sporen in einer Konzentration von 10⁵ KBE/Gramm bis 10¹² KBE/Gramm oder von 10⁵ KBE/ml bis 10¹² KBE/ml vorliegt und wobei eine wirksame Menge der Zusammensetzung ein agronomisch relevantes Merkmal der Pflanze verbessert oder ein eine pilzliche Pflanzenerkrankung verursachendes pilzliches Pflanzenpathogen bekämpft.

2. Zusammensetzung nach Anspruch 1, wobei die Zusammensetzung (a) eine Zellpaste oder (b) ein Spritzpulver oder (c) eine sprühgetrocknete Formulierung oder (d) eine stabile Formulierung umfasst.

3. Isolierte biologisch reine Kultur eines Bakterienstamms, umfassend:
(a) AIP61892 oder
(b) eine Spore oder eine Vorspore oder eine Kombination von Zellen, Vorsporen und/oder Sporen von AIP61892,
wobei der Bakterienstamm vorzugsweise resistent gegen ein aus einem Herbizid, einem Fungizid, einem Pestizid oder einer Pflanzenschutzchemikalie ausgewähltes Biozid ist, wobei die Kultur durch Kultivieren in Gegenwart des Biozids hergestellt wird, wobei der Bakterienstamm ein eine pilzliche Pflanzenerkrankung verursachendes pilzliches Pflanzenpathogen bekämpft.

4. Isolierte biologisch reine Kultur nach Anspruch 3, wobei die biologisch reine Kultur in der Lage ist, in Gegenwart von Glyphosat zu wachsen.

5. Bakterienkultur, umfassend
(a) AIP61892 oder
(b) eine Spore oder eine Vorspore oder eine Kombination von Zellen, Vorsporen und/oder Sporen von AIP61892,
wobei die Bakterienkultur eine antipathogene Aktivität gegen ein eine pilzliche Pflanzenerkrankung verursachendes pilzliches Pflanzenpathogen, das in der Lage ist, in Gegenwart von Glufosinat zu wachsen, aufweist oder eine wirksame Menge der Bakterienkultur ein agronomisch relevantes Merkmal der Pflanze verbessert.

6. Verfahren zum Züchten einer für eine pilzliche Pflanzenerkrankung anfälligen Pflanze oder zum Verbessern eines agronomisch relevanten Merkmals in einer Pflanze, umfassend das Ausbringen
(a) einer wirksame Menge des Bakterienstamms AIP61892 und/oder
(b) einer wirksame Menge einer Spore und/oder einer Vorspore und/oder einer Kombination von Zellen, Vorsporen und/oder Sporen aus AIP61892
auf die Pflanze, wobei die wirksame Menge mindestens 10¹² bis 10¹⁶ koloniebildende Einheiten (KBE) pro Hektar umfasst und wobei die wirksame Menge ein die pilzliche Pflanzenerkrankung verursachendes pilzliche Pflanzenpathogen bekämpft oder das relevante agronomische Merkmal verbessert.

7. Verfahren nach Anspruch 6, wobei das Verfahren den Ertrag der für die pilzliche Pflanzenerkrankung anfälligen Pflanze erhöht.

8. Verfahren nach Anspruch 6 oder 7, wobei die pilzliche Pflanzenerkrankung asiatischer Sojabohnenrost (Asian Soybean Rust, ASR) oder Keimlingsfäule ist.

9. Verfahren nach einem der Ansprüche 6-8, wobei das pilzliche Pflanzenpathogen ein oder mehrere aus der aus Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax und Monilinia fructigena bestehenden Gruppe ausgewählte pilzliche Pathogene umfasst,
wobei das pilzliche Pflanzenpathogen besonders bevorzugt ein oder mehrere aus der aus Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi und Phakopsora meibomiae bestehenden Gruppe ausgewählte pilzliche Pathogene umfasst.

10. Verfahren zur Bekämpfung eines eine pilzliche Pflanzenerkrankung in einem Anbaugebiet verursachenden pilzlichen Pflanzenpathogens, umfassend:
(a) das Bepflanzen des Anbaugebiets mit für die pilzliche Pflanzenerkrankung anfälligen Samen oder Pflanzen und
(b) das Ausbringen, auf die für die pilzliche Pflanzenerkrankung anfällige Pflanze, einer wirksamen Menge mindestens eines Bakterienstammes, umfassend
i. AIP61892 oder
ii. eine Spore oder eine Vorspore oder eine Kombination von Zellen, Vorsporen und/oder Sporen von AIP61892, wobei die wirksame Menge mindestens 10⁵ bis 10¹⁶ koloniebildende Einheiten (KBE) pro Hektar umfasst.

11. Verfahren nach Anspruch 10, wobei die Pflanze anfällig gegenüber asiatischem Sojabohnenrost (Asian Soybean Rust, ASR) oder Keimlingsfäule ist.

12. Verfahren nach Anspruch 10 oder 11, wobei das pilzliche Pflanzenpathogen ein oder mehrere aus der aus Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax und Monilinia fructigena bestehenden Gruppe ausgewählte pilzliche Pathogene umfasst,
wobei das pilzliche Pflanzenpathogen besonders bevorzugt ein oder mehrere aus der aus Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi und Phakopsora meibomiae bestehenden Gruppe ausgewählte pilzliche Pathogene umfasst.

13. Verfahren nach einem der Ansprüche 10-12, wobei das Verfahren weiterhin das Ausbringen einer wirksamen Menge eines Biozids umfasst, wobei die wirksame Menge des Biozids selektiv einen relevanten Organismus bekämpft, während die Kultur nicht signifikant geschädigt wird, wobei der Bakterienstamm und das Biozid vorzugsweise gleichzeitig ausgebracht werden oder
wobei der Bakterienstamm und das Biozid vorzugsweise nacheinander ausgebracht werden.

14. Verfahren nach Anspruch 13, wobei das Biozid ein Fungizid ist.

15. Verfahren zur Herstellung eines modifizierten Bakterienstamms mit erhöhter Resistenz gegenüber einem relevanten Biozid, wobei das Verfahren Folgendes umfasst:
(a) die Bereitstellung einer Population eines AIP61892 umfassenden Bakterienstamms, wobei der Bakterienstamm gegenüber dem relevanten Biozid anfällig ist,
(b) das Kultivieren des Bakterienstamms in Gegenwart des relevanten Biozids und
(c) das Auswählen eines modifizierten Bakterienstamms mit einer erhöhten Resistenz gegenüber dem relevanten Biozid, wobei der modifizierte Bakterienstamm die Fähigkeit beibehält, eine oder mehrere pilzliche Pflanzenkrankheiten zu bekämpfen und/oder ein oder mehrere pilzliche Pflanzenpathogene zu bekämpfen.

16. Verfahren nach Anspruch 15, wobei das Kultivieren das Erhöhen der Konzentration des Biozids über die Zeit umfasst.

17. Verfahren nach Anspruch 15 oder 16, wobei das Biozid Glyphosat oder Glufosinat ist.

18. Verfahren zur Behandlung oder Prävention einer pilzliche Pflanzenerkrankung, umfassend das Ausbringen einer wirksamen Menge eines Bakterienstammes auf eine Pflanze mit einer pilzliche Pflanzenerkrankung oder mit dem Risiko, eine pilzliche Pflanzenerkrankung zu entwickeln, umfassend:
(a) AIP61892 und/oder
(b) eine Spore und/oder eine Vorspore und/oder eine Kombination von Zellen, Vorsporen und/oder Sporen von AIP61892,
wobei die wirksame Menge mindestens 10¹² bis 10¹⁶ KBE pro Hektar umfasst und wobei der Bakterienstamm ein die pilzliche Pflanzenerkrankung verursachendes pilzliche Pflanzenpathogen bekämpft.

19. Verfahren nach Anspruch 18, wobei der Bakterienstamm eine oder mehrere pilzliche Pflanzenkrankheiten behandelt oder verhindert, wobei die eine oder die mehreren pilzlichen Pflanzenkrankheiten vorzugsweise asiatischen Sojabohnenrost (Asian Soybean Rust, ASR) oder Keimlingsfäule umfassen.

20. Verfahren nach Anspruch 18 oder 19, wobei der Bakterienstamm ein oder mehrere pilzliche Pflanzenpathogene bekämpft.

21. Verfahren nach Anspruch 20, wobei das eine oder die mehreren pilzlichen Pflanzenpathogen aus der aus Botrytis cinerea, Cersospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax und Monilinia fructigena bestehenden Gruppe ausgewählt sind,
wobei das ein oder die mehreren pilzlichen Pflanzenpathogen besonders bevorzugt aus der aus Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi und Phakopsora meibomiae bestehenden Gruppe ausgewählt sind.

## Revendications

1. Composition comprenant :
(a) une souche bactérienne AIP61892 ; et/ou
(b) au moins l'une d'une spore ou d'une préspore ou d'une combinaison de cellules, de préspores et/ou de spores issues de AIP61892 ;
dans laquelle ladite souche bactérienne, spore ou préspore ou combinaison de cellules, préspores et/ou spores est présente à hauteur de 10⁵ UFC/gramme à 10¹² UFC/gramme ou à hauteur de 10⁵ UFC/ml à 10¹² UFC/ml et dans laquelle une quantité efficace de ladite composition améliore un caractère agronomique d'intérêt de la plante ou lutte contre un pathogène fongique des plantes qui provoque une maladie fongique des plantes.

2. Composition selon la revendication 1, ladite composition comprenant (a) une pâte cellulaire ou (b) une poudre mouillable ou (c) une formulation séchée par pulvérisation ou (d) une formulation stable.

3. Culture biologiquement pure isolée d'une souche bactérienne comprenant :
(a) AIP61892 ; ou
(b) une spore ou une préspore ou une combinaison de cellules, de préspores et/ou de spores issues de AIP61892, de préférence,
dans laquelle ladite souche bactérienne est résistante à un biocide choisi parmi un herbicide, un fongicide, un pesticide ou un produit chimique de protection des récoltes, ladite culture étant produite par croissance en présence dudit biocide, dans laquelle ladite souche bactérienne lutte contre un pathogène fongique des plantes qui provoque une maladie fongique des plantes.

4. Culture biologiquement pure isolée selon la revendication 3, ladite culture biologiquement pure étant capable de croître en présence de glyphosate.

5. Culture bactérienne comprenant
(a) AIP61892 ; ou
(b) une spore ou une préspore ou une combinaison de cellules, de préspores et/ou de spores issues de AIP61892 ;
ladite culture bactérienne ayant une activité antipathogène contre un pathogène fongique des plantes qui provoque une maladie fongique des plantes et étant capable de croître en présence de glufosinate ou une quantité efficace de ladite culture bactérienne améliorant un caractère agronomique d'intérêt de la plante.

6. Procédé pour faire pousser une plante sensible à une maladie fongique des plantes ou améliorer un caractère agronomique d'intérêt chez une plante, comprenant l'application à la plante
(a) d'une quantité efficace de souche bactérienne AIP61892 ; et/ou
(b) d'une quantité efficace d'au moins l'une d'une spore ou d'une préspore ou d'une combinaison de cellules, de préspores et/ou de spores issues de AIP61892 ;
dans lequel ladite quantité efficace comprend au moins 10¹² à 10¹⁶ unités formant des colonies (UFC) par hectare et dans lequel ladite quantité efficace lutte contre un pathogène fongique des plantes qui provoque la maladie fongique des plantes ou améliore le caractère agronomique d'intérêt.

7. Procédé selon la revendication 6, ledit procédé augmentant le rendement de production de la plante sensible à la maladie fongique des plantes.

8. Procédé selon la revendication 6 ou 7, dans lequel la maladie fongique des plantes est la rouille asiatique du soja (ASR) ou le complexe de fonte des semis.

9. Procédé selon l'une quelconque des revendications 6 à 8, dans lequel le pathogène fongique des plantes comprend un ou plusieurs pathogènes fongiques choisis dans le groupe constitué par Botrytis cinerea, Cercospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax et Monilinia fructigena, plus préférablement,
dans lequel ledit pathogène fongique des plantes comprend un ou plusieurs pathogènes fongiques choisis dans le groupe constitué par Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi et Phakopsora meibomiae.

10. Procédé de lutte contre un pathogène fongique des plantes qui provoque une maladie fongique des plantes dans une zone de culture, comprenant :
(a) la plantation de la zone de culture avec des semences ou des plants sensibles à la maladie fongique des plantes ; et
(b) l'application à la plante sensible à la maladie fongique des plantes d'une quantité efficace d'au moins une souche bactérienne comprenant
i. AIP61892 ; ou
ii. une spore ou une préspore ou une combinaison de cellules, de préspores et/ou de spores issues de AIP61892,
dans lequel ladite quantité efficace comprend au moins 10⁵ à 10¹⁶ unités formant des colonies (UFC) par hectare.

11. Procédé selon la revendication 10, dans lequel ladite plante est sensible à la rouille asiatique du soja (ASR) ou un complexe de fonte des semis.

12. Procédé selon la revendication 10 ou 11, dans lequel le pathogène fongique des plantes comprend un ou plusieurs pathogènes fongiques choisis dans le groupe constitué par Botrytis cinerea, Cercospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax et Monilinia fructigena, plus préférablement,
dans lequel le pathogène fongique des plantes comprend un ou plusieurs pathogènes fongiques choisis dans le groupe constitué par Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi et Phakopsora meibomiae.

13. Procédé selon l'une quelconque des revendications 10 à 12, ledit procédé comprenant en outre l'application d'une quantité efficace d'un biocide, ladite quantité efficace du biocide luttant sélectivement contre un organisme d'intérêt tout en n'endommageant pas significativement la récolte, de préférence,
la souche bactérienne et le biocide étant appliqués simultanément ou
la souche bactérienne et le biocide étant appliqués séquentiellement.

14. Procédé selon la revendication 13, dans lequel le biocide est un fongicide.

15. Procédé de préparation d'une souche bactérienne modifiée ayant une résistance accrue à un biocide d'intérêt, le procédé comprenant :
(a) l'utilisation d'une population d'une souche bactérienne comprenant AIP61892, ladite souche bactérienne étant sensible au biocide d'intérêt ;
(b) la culture de ladite souche bactérienne en présence du biocide d'intérêt ; et
(c) la sélection d'une souche bactérienne modifiée ayant une résistance accrue audit biocide d'intérêt, la souche bactérienne modifiée conservant la capacité à lutter contre une ou plusieurs maladies fongiques des plantes et/ou à lutter contre un ou plusieurs pathogènes fongiques des plantes.

16. Procédé selon la revendication 15, dans lequel ladite culture comprend l'augmentation de la concentration du biocide au cours du temps.

17. Procédé selon la revendication 15 ou 16, dans lequel ledit biocide est le glyphosate ou le glufosinate.

18. Procédé de traitement ou de prévention d'une maladie fongique des plantes comprenant l'application à une plante ayant une maladie fongique des plantes ou présentant un risque de développer une maladie fongique des plantes d'une quantité efficace d'une souche bactérienne comprenant :
(a) AIP61892 ; et/ou
(b) au moins l'une d'une spore ou d'une préspore ou d'une combinaison de cellules, de préspores et/ou de spores issues de AIP61892,
dans lequel ladite quantité efficace comprend au moins 10¹² à 10¹⁶ UFC par hectare et dans lequel ladite souche bactérienne lutte contre un pathogène fongique des plantes qui provoque la maladie fongique des plantes.

19. Procédé selon la revendication 18, dans lequel la souche bactérienne traite ou prévient une ou plusieurs maladies fongiques des plantes, de préférence, dans lequel la ou les maladies fongiques des plantes comprennent la rouille asiatique du soja (ASR) ou un complexe de fonte des semis.

20. Procédé selon la revendication 18 ou 19, dans lequel la souche bactérienne lutte contre un ou plusieurs pathogènes fongiques des plantes.

21. Procédé selon la revendication 20, dans lequel le ou les pathogènes fongiques des plantes sont choisis dans le groupe constitué par Botrytis cinerea, Cercospora spp, Cercospora sojina, Cercospora beticola, Alternaria solani, Rhizoctonia solani, Blumeria graminis f. sp. Tritici, Erysiphe necator, Podosphaera xanthii, Golovinomyces cichoracearum, Erysiphe lagerstroemiae, Sphaerotheca pannosa, Colletotrichum cereale, Apiognomonia errabunda, Apiognomonia veneta, Colletotrichum gloeosporiodes, Discula fraxinea, Plasmopara viticola, Pseudoperonospora cubensis, Peronospora belbahrii, Bremia lactucae, Peronospora lamii, Plasmopara obduscens, Pythium cryptoirregulare, Pythium aphanidermatum, Pythium irregulare, Pythium sylvaticum, Pythium myriotylum, Pythium ultimum, Phytophthora capsici, Phytophthora nicotianae, Phytophthora infestans, Phytophthora tropicalis, Phytophthora sojae, Fusarium graminearum, Fusarium solani, Fusarium oxysporum, Fusarium graminicola, Gibberella zeae, Colletotrichum graminicola, Phakopsora sp., Phakopsora meibomiae, Phakopsora pachyrizi, Puccinia triticina, Puccinia recondita, Puccinia striiformis, Puccinia graminis, Puccinia spp., Venturia inaegualis, Verticillium spp, Monilinia fructicola, Monilinia lax et Monilinia fructigena, plus préférablement,
dans lequel le ou les pathogènes fongiques sont choisis dans le groupe constitué par Botrytis cinerea, Cercospora sojina, Alternaria solani, Rhizoctonia solani, Erysiphe necator, Podosphaera xanthii, Plasmopara viticola, Peronospora belbahrii, Pythium aphanidermatum, Pythium sylvaticum, Pythium myriotylum, Pythium ultimum, Phytophthora nicotianae, Phytophthora infestans, Phytophthora tropicalis, Phytophthora sojae, Fusarium graminearum, Fusarium solani, Phakopsora pachyrizi et Phakopsora meibomiae.
